# EUROPEAN PATENT SPECIFICATION

(11) **EP 1 613 743 B1**
(45) Date of publication and mention of the grant of the patent: **18.11.2009**
(21) Application number: 04715201.2
(22) Date of filing: 27.02.2004
(51) Int. Cl.: C12N 5/08, G01N 9/30, C07K 16/18, C07K 16/28

(54) **FETAL CELL ISOLATION AND ENRICHMENT**
ISOLIERUNG UND ANREICHERUNG FÖTALER ZELLEN
ISOLEMENT ET ENRICHISSEMENT DE CELLULES FOETALES

(30) Priority: 28.02.2003 AU 2003900944
(43) Date of publication of application: 11.01.2006
(73) Proprietor: THE UNIVERSITY OF QUEENSLAND, St Lucia, QLD 4072 (AU)
(72) Inventor: IRWIN, Darryl, Luke, Grange, Queensland 4051 (AU); FINDLAY, Ian, Albany Creek, Queensland 4035 (AU)
(74) Representative: Elsy, David
(86) International application number: PCT/AU2004/000248
(87) International publication number: WO 2004/076653

(56) References cited:
- WO-A1-03/020986
- WO-A1-03/102595
- US-A- 5 676 849
- TUTSCHEK B ET AL: "ISOLATION OF FETAL CELLS FROM TRANSCERVICAL SAMPLES BY MICROMANIPULATION: MOLECULAR CONFIRMATION OF THEIR FETAL ORIGIN AND DIAGNOSIS OF FETAL ANEUPLOIDY" PRENATAL DIAGNOSIS, CHICHESTER, SUSSEX, GB, vol. 15, no. 10, October 1995 (1995-10), pages 951-960, XP009051380 ISSN: 0197-3851
- SCHUELER P A ET AL: "Enrichment and identification of fetal trophoblast cells from first trimester maternal cervical lavage and uterine blood specimens" PLACENTA, W.B. SAUNDERS, vol. 22, no. 8-9, September 2001 (2001-09), pages 688-701, XP002351396 ISSN: 0143-4004
- FINDLAY I ET AL: "USING MF-PCR TO DIAGNOSE MULTIPLE DEFECTS FROM SINGLE CELLS: IMPLICATIONS FOR PGD" MOLECULAR AND CELLULAR ENDOCRINOLOGY, AMSTERDAM, NL, vol. 183, 2001, pages S05-S12, XP001199454 ISSN: 0303-7207
- YAMANISHI D.T. ET AL.: 'Enrichment of rare fetal cells from maternal peripheral blood' EXPERT REVIEW OF MOLECULAR DIAGNOSTICS vol. 2, no. 4, 2002, pages 303 - 311, XP008054672
- ZHAO X.X. ET AL.: 'Enrichment of fetal cells from maternal blood by magnetic activated cell sorting (MACS) with fetal cell specific antibodies:one-step versus two-step MACS' COGENITAL ANOMALIES vol. 42, no. 2, 2002, pages 120 - 124, XP008081661
- HOLZGREVE W. ET AL.: 'Fetal cells in cervical mucus and maternal blood' BAILLIERE'S CLINICAL OBSTETRICS AND GYNECOLOGY vol. 14, no. 4, 2000, pages 709 - 722, XP008081646
- MILLER D. ET AL.: 'Transcervical recovery of fetal cells from the lower uterine pole:reliability of recovery and histological/immunocytochemical analysis of recovered cell population' HUMAN REPRODUCTION vol. 14, no. 2, 1999, pages 521 - 531, XP002986610
- CIRIGLIANO V. ET AL: 'Transcervical cells and the prenatal diagnosis of hemoglobin (Hb) mutations' CLINICAL GENETICS vol. 56, no. 5, 1999, pages 358 - 362, XP003013572

## Description

THIS INVENTION relates to isolation and enrichment of cells from cervical samples. More particularly, this invention relates to enrichment of fetal cells from cervical samples, and in particular, Pap smears. In a particular form, this invention relates to use of fetal cells enriched from cervical samples for subsequent genetic analysis.

### BACKGROUND OF THE INVENTION

Currently prenatal diagnosis of chromosomal and single gene disorders requires the extraction of fetal cells from the uterine cavity by invasive procedures such as amniocentesis or chorionic villus sampling (CVS). These techniques, although highly reliable, carry procedurally related risks such as miscarriage (0.5-1%), require a high level of technical expertise, take several weeks for results and can only be performed relatively late in pregnancy. Thus, they are only offered to women considered at high risk due to age, genetic history or other indicative factors.

One less invasive alternative approach is to use maternal blood as a source of fetal cells for which fetal cell enrichment methods have been developed, for example as described in United States Patent 5,629,147, United State Patent 5,646,004 and International Publication WO 93/02528.

However major technical difficulties remain due to the extremely low numbers of fetal cells found in the maternal circulation, the extreme difficulties in isolating such cells, the positive identification of fetal cells and the presence of fetal cells from previous pregnancies which may confound identification and diagnosis.

The presence of fetal cells in the endocervical canal was first published in Shettes, 1971, Nature 230 52. Since then studies have confirmed the presence of fetal cells during the first trimester (Fejgin et al., 2001, Prenatal Diagnosis 21 619) and that the number of fetal cells present in the endocervical canal of pregnant women is extremely low and are difficult to isolate.

Accordingly, the value of maternal cervical samples as a source of fetal cells for genetic analysis has remained controversial. Additionally, there have been major concerns as to the invasiveness and safety of cervical sampling and the practicality of using cervical samples as a source of relatively low abundance fetal cells.

Indeed, Overton et al., 1996, J. Am. Obstet. Gynecol. 175 382 concluded that fetal cells cannot be obtained from the endocervix by minimally invasive techniques in sufficient yield for prenatal genetic diagnosis.

More recently Cioni et al., 2003 Prenatal Diagnosis 23 168-171 confirmed that fetal cells were not detected in a consistent and reliable fashion and therefore such sampling techniques cannot be regarded as a promising tool towards minimally invasive prenatal diagnosis.

### SUMMARY OF THE INVENTION

Notwithstanding the prior art teaching that cervical samples are very poor sources of fetal cells for genetic analysis, the present inventors have surprisingly developed a reliable and highly efficient method of isolating and enriching fetal cells from cervical samples. More particularly, the present invention has for the first time utilized Pap smears, which is a particularly safe cervical sampling method for obtaining fetal cells suitable for enrichment and subsequent analysis.

The invention therefore broadly relates to the isolation and enrichment of fetal cells from cervical samples such as Pap smears.

Therefore, in one aspect, the invention provides a method of fetal cell enrichment from a cervical sample that includes at least one step selected from:
(i) enriching one or more fetal cells according to density;
(ii) enriching one or more fetal cells by charge flow separation; and/or
(iii) enriching one or more fetal cells by differential cell lysis; and at least one further step of:
(iv) enriching one or more fetal cells by negatively selecting maternal cells using at least one antibody that binds a maternal cell antigen; and/or
(v) enriching one or more fetal cells by positively selecting said one or more fetal cells using at least one antibody that binds a fetal cell antigen.

In another aspect, the invention provides a method of fetal cell enrichment from a Pap smear that includes at least one step selected from:
(i) enriching one or more fetal cells according to density;
(ii) enriching one or more fetal cells by charge flow separation; and/or
(iii) enriching one or more fetal cells by differential cell lysis;
(iv) enriching one or more fetal cells by negatively selecting maternal cells using at least one antibody that binds a maternal cell antigen; and/or
(v) enriching one or more fetal cells by positively selecting said one or more fetal cells using at least one antibody that binds a fetal cell antigen.

In a preferred embodiment, the invention provides a method of fetal cell enrichment including the steps of
(A) enriching one or more fetal cells according to density using a preformed discontinuous density gradient;
(B) enriching one or more fetal cells from step (A) that express a first fetal cell antigen; and
(C) further enriching the one or more fetal cells from step (B) that express a second fetal cell antigen.

In a further aspect, the invention provides a method of obtaining a nucleic acid sample, including the step of isolating a nucleic acid from one or more fetal cells enriched according to any one of the aforementioned aspects.

In a still further aspect, the invention provides a method of nucleic acid sequence amplification including the steps of:
(i) isolating a nucleic acid according to the aforementioned aspect; and
(ii) subjecting the nucleic acid isolated at step (i) to a nucleic acid sequence amplification technique.

In a still yet further aspect, the invention provides a method of genetic analysis including the step of analyzing a nucleic acid obtained from one or more fetal cells according to the preceding aspect.

It will also be appreciated that other analyses also contemplated by the present invention include biochemical analysis, morphological analysis, histology, cytology, cell culture and the like.

The invention also relates to the automation of enrichment steps described herein to enrich fetal material from cervical samples.

Preferably, the cervical sample is obtained using an endocervical brush or cytobrush.

More preferably, the cervical sample is a Pap smear.

Throughout this specification, unless otherwise indicated, "comprise", "comprises" and "comprising" are used inclusively rather than exclusively, so that a stated integer or group of integers may include one or more other non-stated integers or groups of integers.

### BRIEF DESCRIPTION OF THE FIGURES AND TABLES

Table 1. Gestation and percentage of fluorescent cells in the untreated sample.
Table 2: Gestation of each patient, the percentage of cells with a high level of fluorescence (++) and the overall percentage of cells exhibiting fluorescence higher than the background for each antibody set (A and B). Also shown is the number of high level fluorescent cells isolated, the total number of cells isolated with fluorescence higher than background levels, the percentage of both these cell sets that exhibited a fetal signal. The number of cells on the slide has been extrapolated to indicate the overall number of cells in the sample. The percentage fluorescence and percentage of these that exhibit a fetal fingerprint have been extrapolated to show the number of fetal cells within the sample.
Table 3. Specificity of each antibody set towards fetal cells. Antibody set A is PLac and LK26, Antibody set B is HCG and PLAP.
Table 4: Patient gestation (weeks), macroscopic observations noting the presence of blood, number of cells and cell clumping (- Not visible, + Low, ++ Moderate, +++ High).
Table 5: Percentage of fetal cells in initial sample and density gradient enriched sample identified using immunohistochemistry. Percentage of fetal cells and number of cells analyzed after antibody enrichment, identified by MFPCR. Fold enrichment and percentage fetal cell loss compared to unprocessed sample for each stage.
Table 6: Comparison of simultaneous, continuous gradient centrifugation with non-simultaneous (pre-formed), continuous gradient centrifugation. Frequency of fetal cells was determined by double positive immunohistochemistry.
Table 7: Comparison of simultaneous, continuous gradient centrifugation with non-simultaneous (pre-formed), discontinuous gradient centrifugation and evaluation of repeating gradient centrifugation on fetal cell enrichment. Frequency of fetal cells was determined by double positive immunohistochemistry.
Table 8: Evaluation of sensitivity and specificity of anti-epithelial antibodies towards cells present in Pap smears using a double positive immunohistochemistry method with anti human placental lactogen.
Figure 1: Density of gradient bands using density marker beads.
Figure 2: Percentage of fetal cells in each gradient isolation.
Figure 3: Numbers of fetal cells in each gradient.
Figure 4: Representative photographs of Low level fluorescence for FITC (Top left) and Rhodamine (Top right) as well as High level fluorescence for FITC (Bottom left) and Rhodamine (Bottom right).
Figure 5A: Percentage of fluorescent cells compared to the gestation of the pregnancy for antibodies to PLac and LK26.
Figure 5B: Percentage of fluorescent cells compared to the gestation of the pregnancy for antibodies to HCG and FLAP.
Figure 6A Number of fluorescent cells in each sample compared to the gestation of the pregnancy for antibodies to PLac and LK26.
Figure 6B: Number of fluorescent cells in each sample compared to the gestation of the pregnancy for antibodies to HUG and FLAP.
Figure 7: Electrophoretic DNA fingerprinting profiles from maternal (top), fetal (middle) and mixed maternal/fetal (bottom) samples. This demonstrates absolute identification of the isolated cell as fetal, rather than maternal, in origin
Figure 8: Representative electropherograms of the maternal buccal cells and predicted isolated fetal cells. For each numbered Pap smear sample, the fetal cell samples are shown in the "top" panel while maternal cell samples are shown in the "bottom" panel.
Figure 9: Comparison of fetal cell fold-enrichment using a single and double gradient centrifugation step. Frequency of fetal cells was determined by double positive immunohistochemistry. Step 1: Gradient enrichment; Step 2: First MACS enrichment; Step 3: Second MACS enrichment.

### DETAILED DESCRIPTION OF THE INVENTION

The present invention provides a variety of methods applicable to enrichment of fetal cells from maternal cervical samples, and in particular, from Pap smears. Such methods include percoll density gradient centrifugation, use of antibodies to human placental lactogen and folate binding protein and magnetic activated cell sorting (MACS), this latter method selecting fetal cells with a minimum of 54% specificity as confirmed by MFPCR.

It will also be appreciated that the present invention is applicable to isolation or enrichment of other cells of non-maternal origin including, but not limited to, embryonic cells, sperm cells and any cells of cytotrophoblast, cytiotrophoblast, syncytiotrophoblast or villus mesenchyme origin.

For the purposes of this invention, by "*isolated*" is meant material that has been removed from its natural state or otherwise been subjected to human manipulation. Isolated material may be substantially or essentially free from components that normally accompany it in its natural state, or may be manipulated so as to be in an artificial state together with components that normally accompany it in its natural state.

By "*enrich*", "*enriched*" and "*enrichment*" in the context of Cell isolation is meant that cells are obtained in a higher frequency of proportion compared to their frequency or proportion in a starting sample prior to enrichment. In this context enrichment is also taken to include 100% enrichment where the fetal cell or cells exist in the absence of maternal cells, such as would apply to the isolation of single fetal cells.

In a preferred embodiment, the method of the invention provides at least 10-fold, more preferably at least 20-fold, even more preferably at least 50-fold and advantageously at least 100-fold to 150-fold enrichment of fetal cells from a cervical sample such as a Pap smear.

Suitably, fetal cells are enriched from a cervical sample. Such samples include and encompass any sample obtained from the endocervix inclusive of endocervical lavage, aspiration, swabbing; cytobrush samples (e.g. Pap smears); and transcervical samples (TCCs).

Preferably, the cervical sample is a Pap smear.

As used herein, a Pap smear is a biological sample comprising one or more cells collected or obtained as a scraping from the cervix.

Typically, a metal or plastic instrument such as a speculum is placed in the vagina to allow visualization of the interior of the vagina and the cervix. A sampling instrument such as a small wooden spatula or cytobrush is used to scrape the outside of the cervix and thereby obtain the cervical sample.

For the purposes of cervical cancer screening, the scrapings are placed on a glass slide and used for microscopic examination to detect changes in the cells of the cervix. Pap smears are a routine and safe screening procedure to find early warning signs of cervical cancer. The present invention provides a new use of Pap smears as a source of fetal cells for enrichment and subsequent analysis.

In the context of the present invention, said one or more cells typically comprises maternal cells and fetal cells.

For the particular purpose of fetal cell isolation, it is preferred that the Pap smear is obtained at between 5 and 31 weeks gestation (*i*.*e* the number of weeks since the last menstrual period).

Cell enrichment may be performed by one or more cell isolation methods including but not limited to differential lysis, density gradient separation, micromanipulation, complement-mediated lysis, flow cytometry, magnetic bead separation, panning, charge flow separation and cell culture methods that promote selective propagation of cells to be enriched.

Each cell enrichment method may be performed alone or in combination with one ar more other methods to thereby achieve a desired level of cell enrichment or purity.

Additional treatments may be utilized that facilitate cell isolation and enrichment, for example in one embodiment protease treatment (*e*.*g*. trypsin digestion) of cervical samples may be performed prior to density gradient enrichment.

### Gradient Centrifugation

Density gradients may be used to enrich fetal cells from cervical samples, either as a single-step or multi-step procedure.

Density gradients may be continuous or discontinuous and may be formed using media such as Metrizamide^{™}, Ficoll^{™} and Percoll^{™}, although without imitation thereto.

Preferably, fetal cells are enriched using a Percoll^{™} density gradient.

Furthermore, formation of the density gradient may be preformed before density enrichment of the cell sample (*i*.*e* non-simultaneously), or simultaneously with density enrichment of the cells.

Typically fetal cells are enriched by virtue of having a greater density than that of contaminating maternal cells.

Generally, fetal cells may be enriched in a density fraction in the range 1.033-1.142 g/mL

Accordingly, maternal cells are typically enriched in a 1.018-1.033 g/mL density fraction.

In one embodiment, fetal cells are enriched by a continuous density gradient.

Preferably, fetal cells are enriched in any density fraction in the range 1.049-1.142 g/mL.

More preferably, fetal cells are enriched in any density fraction in the range 1.033 to 1.131 g/mL.

In another embodiment, fetal cells are enriched in a discontinuous density gradient.

In a preferred form of this embodiment, density gradient separation is by non-simultaneous discontinuous density gradient enrichment.

According to a particularly preferred form of this embodiment, fetal cells may be more highly enriched in a density fraction in the range 1.05 to 1.131 g/mL.

Preferably, according to the aforementioned embodiments density gradient enrichment is performed using sequential density gradient separation steps.

It will also be appreciated that in embodiments where sequential enrichment procedures are used, it may be advantageous to initially obtain a broader density gradient fraction to ensure a high yield, the purity of which is increased by one or more subsequent enrichment steps.

Indeed, as will be described in detail hereinafter, sequential density gradient enrichment steps increase fetal cell fold-enrichment compared to a single density gradient enrichment step.

### Differential Lysis

Differential lysis exploits physical properties of cell membranes and, more particularly, cellular susceptibility to lysis in conditions different to the normal extracellular environment.

In one embodiment, the invention contemplates differential lysis of maternal red blood cells (RBCs) which may be a contaminant in cervical samples using a hypotonic solution (commonly 0.075M KC1) leaving nucleated cells intact. Another method of RBC lysis is using the hypotonic solution 155mM ammonium chloride/10mM sodium bicarbonate/0.05mM EDTA, lysis occurring due to carbonic anhydrase enzyme activity.

Of relevance to enrichment of fetal NRBCs, it has also been shown that fetal NRBCs are less sensitive to NH₄Cl/HCO₃⁻ mediated lysis because of their lower carbonic anhydrase (CA) activity (Boyer et al., Blood 47 8S3-897). Therefore fetal RBCs may be protected from lysis by using an inhibitor of CA (Acetazolamide) at a concentration that completely blocks fetal, but not adult, CA activity. This method has been shown to lyse >99.9% of adult erythrocytes whilst leaving >25% of fetal NRBC's intact (Collarini et al., 2001, Cytometry 45 267-276).

In a particular embodiment, it is contemplated that differential lysis would be utilized where there is substantial contamination of a cervical sample or Pap smear by maternal RBCs. For example, the level of contamination would be at least 5%, preferably at least 10%, more preferably at least 25% and advantageously where maternal RBCs constitute at least 50% of the total cell number present in the sample.

In another embodiment, the invention contemplates complement-mediated lysis of maternal cells, particularly maternal epithelial cells. Complement-mediated lysis uses one or more antibodies which selectively bind a respective maternal cell antigen, while preferably displaying minimal or absent cross-reactivity with fetal cells, to thereby "fix" complement which is added to the cell sample.

Complement may typically be in the form of an animal serum fraction (e.g. rabbit, rat or mouse complement fraction) or in the form of reconstituted lyophilized complement.

Appropriate incubation of a cervical sample in the presence of complement-fixing antibody together with complement will selectively lyse and kill maternal cells with minimal, or at least tolerable, non-specific loss of fetal cells resulting in fetal cell enrichment.

Non-limiting examples of human epithelial cell antigens and antibodies thereto which are contemplated by the present invention include Epithelial Membrane Antigen (EMA); Epithelial Specific Antigen (ESA); Cytokeratin (AE1/AE3); Cytokeratin 7; Cytokeratin 20; Cytokeratin S, 18, 19 (Low molecular weight)(CAM 5.2); Cytokeratin 1,2,5,10,14/15 (High Molecular Weight) (Clone 34BE12); Cytokeratin 10,17,18 (MNF-116); CEA (clone Col 1); Estrogen Receptor; BCL-2 and Ham56

### Antibody-based enrichment

It will be appreciated that fetal cell enrichment may be achieved using antibodies directed to fetal antigens not expressed, or expressed at low levels, by maternal cells.

Alternatively, fetal cells may be enriched by virtue of their non-expression or low expression of maternal or non-fetal antigens.

Accordingly, fetal cell enrichment may be performed by negative depletion of maternal cells and/or positive selection of fetal cells according to antigen expression.

Antigens that may be applicable to antibody-based enrichment include, but are not limited to, glycophorin A, CD36, Fk1-1, EPO-R, human chorionic gonadotrophin (HCG), human placental alkaline phosphatase, human placental lactogen (FD0202N), folate binding protein (LK26) and HLA antigens such as HLA-Class II, for each of which specific antibodies are readily available.

Particularly preferred fetal antigens are human placental lactogen (FD0202N) and folate binding protein (LK26).

In the broadest sense, antibody-based enrichment may utilize any technique that selects cells (*i*.*e* positive selection) or depletes cells (*i*.*e* negative selection) according to antigen expression or non-expression, as the case may be. A non-exhaustive list includes panning, complement-mediated lysis, fluorescence-activated cell sorting (FACS) and magnetic activated cell sorting (MACS).

It will also be appreciated that the aforementioned techniques may be used alone or in sequential combination to enrich fetal cells.

For FAGS enrichment, fluorescently-labeled antibodies are bound to the cells of interest. These cells are then passed through the excitation laser in a single cell stream and measured for size, granularity and fluorescent activity. Specific parameters are set and cells that fall within those parameters (*e*.*g*. fluorescence, forward light scatter, side scatter) are collected by a cell sorter.

Preferred methods utilize MACS.

For MACS enrichment, monoclonal antibodies coupled to small magnetic particles are bound to the cells of interest. Using a magnet, the bound cells may be enriched from contaminating cells. Alternatively, contaminating cells may be removed with bound beads in cases where fetal cells are selected on the basis of not expressing a maternal cell antigen.

In a preferred embodiment, fetal cells are enriched by magnetic activated cell sorting (MACS) using antibody to placental lactogen, trypsin release of bound beads followed by MACS enrichment using antibody to folate binding protein (LK26).

### Charge Flow Separation

Charge flow separation uses dielectrophoretic forces which occur on cells when a non-uniform electrical field interacts with field-induced electrical polarization. Depending on the dielectric properties of the cells relative to their suspending medium, these forces can be positive or negative, directing the cells toward strong or weak electrical field regions. Because cells of different types or in distinct biological states have different dielectric properties, differential dielectrophoretic forces can be applied to drive their separation into purified cell populations (Wang et al., 2000. Analytical Chemistry 72 832-839).

### Cell Culture

It will also be appreciated that fetal cells may be enriched by selective growth in the presence of appropriate cytokines and culture conditions that favor the selective proliferation of fetal progenitor cells over maternal cells. Selective growth may be performed after initial isolation or enrichment by one or more other enrichment methods.

For example, fetal nRBC's may be cultured after gradient enrichment and/or MACS enrichment in culture media containing many fetal NRBC growth factors (Bohmer et al., 1998, Br J Haematol 103 351-360). It is also contemplated that culture with fetal NRBC growth factors may stimulate a much higher basal proliferative capacity than mature progenitor cells and that this can be enhanced by addition of cytokine cocktails such as flt-3 ligand and thrombopoetin (Holzgreve et al., 2000, Baillieres Best Pract Res Clin Obstet Gynaecol 14 709-722).

In light of the foregoing, a particularly preferred embodiment of the invention provides a method of fetal cell enrichment including the steps of:
(i) enriching one or more fetal cells according to density using sequential, preformed discontinuous density gradients; and
(ii) positively selecting one or more fetal cells from step (i) by MACS

Preferably, step (ii) includes the sequential steps of:
(a) magnetic activated cell sorting (MACS) using antibody to placental lactogen;
(b) trypsin digestion to release fetal cells from bound beads; and
(c) MACS enrichment of fetal cells obtained in (b) using antibody to folate binding protein (LK26).

### Nucleic acid isolation and Genetic analysis

A preferred use of cells enriched by the method of the invention is for subsequent genetic analysis, biochemical analysis, immunological analysis, morphological analysis, histology, cytology, cell culture and the like.

A more preferred use is for genetic analysis.

As used herein, "*genetic analysis*" and "*genetic diagnosis*" are used interchangeably and broadly cover detection, analysis, identification and/or characterization of isolated genetic material and includes and encompasses terms such as, but not limited to, genetic identification, genetic diagnosis, genetic screening, genotyping, prenatal genetic diagnosis, paternity testing and DNA fingerprinting which are variously used throughout this specification.

The term "*nucleic acid*" as used herein designates single-or doublestranded mRNA, RNA, cRNA, RNAi and DNA inclusive of cDNA, genomic DNA and DNA-RNA hybrids.

A "*polynucleotide*" is a nucleic acid having eighty (80) or more contiguous nucleotides, while an "*oligonucleotide*" has less than eighty (80) contiguous nucleotides.

A "*primer*" is usually a single-stranded oligonucleotide, preferably having 12-50 contiguous nucleotides which, for example, is capable of annealing to a complementary nucleic acid "template" and being extended in a template-dependent fashion by the action of a DNA polymerase such as *Taq* polymerase, RNA-dependent DNA polymerase or Sequenase^{™}.

By "*genetic marker*" or "*marker*" is meant any locus or region of a genome. The genetic marker may be a coding or non-coding region of a genome. For example, genetic markers may be coding regions of genes, non-coding regions of genes such as introns or promoters, or intervening sequences between genes such as those that include polymorphisms, such as single nucleotide polymorphisms(SNPs), tandem repeat sequences, for example satellites, microsatellites, short tandem repeats (STRs) and minisatellites, although without limitation thereto.

A "*probe*" may be a single or double-stranded oligonucleotide or polynucleotide, suitably labeled for the purpose of detecting complementary sequences in Northern or Southern blotting, for example.

Genetic analysis may be performed by any method including, but not limited to, fluorescence *in situ* hybridization (FISH), primed *in situ* synthesis (PRINS) and nucleic acid sequence amplification, preferably in the form of multiplex fluorescent PCR amplification (MFPCR) or methods that employ nucleic acid arrays such as a microarray format.

It will be appreciated that genetic analysis may be performed using microarrays which are particularly useful when analyzing expression or non-expression of multiple genetic markers and mutation detection in multiple genes.

Examples of fluorescent in *situ* hybridization (FISH) and Primed In Situ Synthesis (PRINS) may be found in Findlay et al., 1998, J. Assisted Reproduction & Genetics 15 257.

As used herein, "*multiplex amplification*" or "*multiplex PCR*" refers to amplification of a plurality of genetic markers in a single amplification reaction.

MFPCR has been shown to be a reliable and accurate method for determining sex (Salido et al., 1992, Am. J Human genetics 50 303; Findlay et al., 1994a, Human Reproduction, 9 23; Findlay et al., 1994b, Advances in Gene Technology: Molecular Biology and Human Genetic Disease. Vol 5, page 62. Findlay et al., 1995, Human Reproduction 10 1005-1013; Findlay *et al*., 1998c, *supra*) diagnosing genetic diseases such as cystic fibrosis (Findlay *et al*., 1995, *supra*), detecting chromosomal aneuploidies and in genetic analyses for genetic identification, such as typically referred to as DNA fingerprinting (Findlay et al., 1997, Nature 389 355-356).

With regard to genetic markers for genetic analysis, preferred genetic markers are STR markers. International Application PCT/AU02/01388 provides an extensive array of STR markers and primers together with MFPCR methodology to successfully amplify multiple STR markers from limiting amounts of nucleic acid template.

Although from the foregoing a. preferred method of genetic analysis is PCR, nucleic acid sequence amplification is not limited to PCR.

Nucleic acid amplification techniques are well known to the skilled addressee, and also include ligase chain reaction (LCR) as for example described in Chapter 15 of Ausubel et al. CURRENT PROTOCOLS IN MOLECULAR BIOLOGY (John Wiley & Sons NY, 1995-1999); strand displacement amplification (SDA) as for example described in U.S. Patent No 5,422,252; rolling circle replication (RCR) as for example described in Liu et al., 1996, J. Am. Chem. Soc. 118 1587 and International application WO 92/01813 and by Lizardi et al., in International Application WO 97/19193; nucleic acid sequence-based amplification (NASBA) as for example described by Sooknanan et al.,1994, Biotechniques 17 1077; and Q-β replicase amplification as for example described by Tyagi et al., 1996, Proc. Natl. Acad. Sci. USA 93 5395.

The abovementioned are examples of nucleic acid sequence amplification techniques but are not presented as an exhaustive list of techniques. Persons skilled in the art will be well aware of a variety of other applicable techniques as well as variations and modifications to the techniques described herein.

As used herein, an "*amplification product*" refers to a nucleic acid product generated by a nucleic acid amplification technique.

Although the invention also contemplates use of nucleic acid other than DNA, preferably the nucleic acid is DNA.

More preferably, the nucleic acid is genomic DNA.

Isolation of cellular nucleic acids is well known in the art, although the skilled person is referred to Chapters 2, 3 and 4 of Ausubel et al. CURRENT PROTOCOLS IN MOLECULAR BIOLOGY (John Wiley & Sons NY, 1995-1999), for examples of nucleic acid isolation.

In a particular embodiment, the invention provides genetic analysis of a single, enriched fetal cell, or at least very small numbers of enriched fetal cells (eg. less than 20).

According to this embodiment, a preferred nucleic acid isolation technique comprises an initial Proteinase K digestion step to "strip" proteins such as antibodies bound to the fetal cell surface antigen(s) followed by alkaline lysis (such as using potassium hydroxide) in the presence of a reducing agent (such as dithiothreitol).

Preferred sources of nucleic acids are mammals, preferably humans.

The invention also contemplates genetic analysis of non-human samples such as from cows, sheep, horses, pigs and any other mammal including companion animals, sporting animals and livestock, although without limitation thereto.

So that the invention may be readily understood and put into practical effect, reference is made to the following non-limiting examples.

### EXAMPLES

### EXAMPLE 1

### Gradient enrichment of fetal cells from PAP smears with positive identification using double monoclonal antibody selection

### Materials and Methods

Informed consent was obtained from four pregnant women between 7 and 31 weeks gestation. A pap smear cervix brush (Rovers Medical Devices, Lekstraat, The Netherlands) was inserted through the external os to a maximum depth of two centimeters. The brush was then removed whilst rotating a full turn. The material that was caught on the brush was smeared on a-slide which was used for routine cervical cancer screening.

The remaining material on the brush was included in the study. Cells were washed off the cervix brush into Dulbeccos PBS (Invitrogen, Melbourne Australia). The cells were then spun at 402g in a Sigma 4K15 centrifuge (Sigma, St Louis, USA), the supernatant was poured off and the remaining material transferred to a 1.53mL centrifuge tube. The cells were then spun at 3000rpm in a biofuge pico (Kendro, Ashville, North Carolina), the supernatant was then poured off and the cells were resuspended in 800uL PBS. A buccal swab was also taken from the mother to provide an uncontaminated source of maternal cells.

A 2:1 dilution of Percoll (Amersham Biosciences, Piscataway, New Jersey) in PBS was prepared and 3mL of Percoll/PBS was added to a Quick-Seal centrifugation tube (Beckman Coulter, Fullerton, CA). A 30uL pap smear cell suspension was layered onto the Percoll/PBS. The tube was then centrifuged at 30000rpm (22000g) in a Beckmann ultrafuge (Beckman Coulter, Fullerton, CA) for 30 minutes at room temperature. A 22 gauge syringe needle (Terumo Medical Corporation, Elkton, MD) was used to neatly piece the bottom of the centrifuge tube leaving syringe embedded. The contents of the centrifuge tube were allowed to drain into eppendorf tubes (200uL, sections degreasing in density) using an empty syringe to apply pressure should the needle become blocked. 1mL PBS was then added to each tube and centrifuged at 5000RPM for 5 minutes in a biofuge pico. Supernatant is then poured off with a repeat wash. The contents were then resuspended in 40uL PBS.

Visible band densities were determined by running samples separately with density marker beads (Amersham Biosciences, Piscataway, New Jersey) using manufacturer's protocols as cells could not be collected simultaneously because the beads clogged the collection syringe.

40uL of cell suspension was placed on superfrost plus microscope slides (Menzel Glaser, Beunos Aires, Argentina). The tube was washed using 20uL of PBS to ensure all cells are transferred and spread by gentle rotation. The slides were allowed to dry at room temperature overnight. Slides were then placed through the following protocol:
- 10 minutes in -20°C Methanol (BDH, Dorset, England) then allowed to air dry.
- Rehydrated 2 X 2mins in PBS
- 10 minutes in 10% Donkey serum (Sigma-Aldrich, St. Louis, USA) diluted in PBS
- Donkey serum was removed
- 100uL of each of the following Primary Antibodies, 1/100 dilution in 10% donkey serum (*i*.*e*. final dilution 1/200) was then added.
   ab7816 Rabbit anti-human chorionic gonadotrophin antibody (Abcam, Cambridge, UK) and NCL-PLAP Mouse anti- placental alkaline phosphatase antibody (Novocastra Laboratories, Newcastle, UK).
- Slides were then incubate 90 minutes at room temperature in humidified chamber
- Slides were then washed 3 X 5 minutes in PBS
- 100uL of each following Secondary antibody 1/200 dilution in PBS (ie final dilution 1/400) was then added.
   Donkey FITC polyclonal to rabbit IgG (beam, Cambridge, UK) and Chicken Rhodamine polyclonal to mouse IgG (Abcam, Cambridge, UK).
- Slides were then incubated 45 minutes at room temperature in humidified chamber
- Slides were then washed 2 X 5 minutes PBS
- Slides were then mounted in PBS with no coverslip.

Slides were then scanned under a fluorescent microscope (Nikon, Melville, USA) with FITC and Rhodamine excitation filters. Total numbers of cells on the slide and the number and fluorescence levels of positive fluorescent cells were recorded.

### Results

Table 1 shows the gestation of the patient and the percentage of fluorescent cells in the untreated sample as determined by immunohistochemistry.

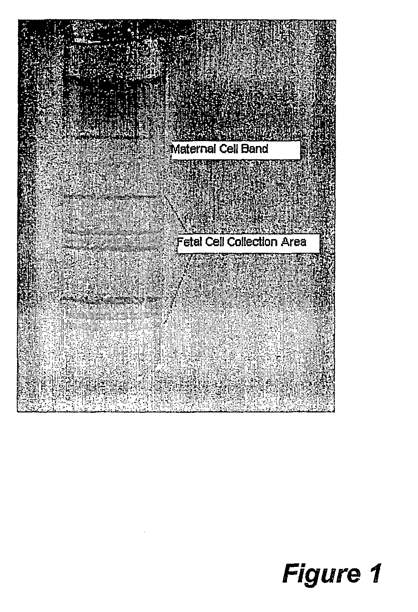

The density of the gradient bands isolated was determined in parallel using density marker beads (Figure 1). Maternal cell band fell between the blue and orange bands, indicating a density of 1.018-1.033g/mL. Fetal cell collection area was between Green and Violet bands (1.049-1.142g/mL).

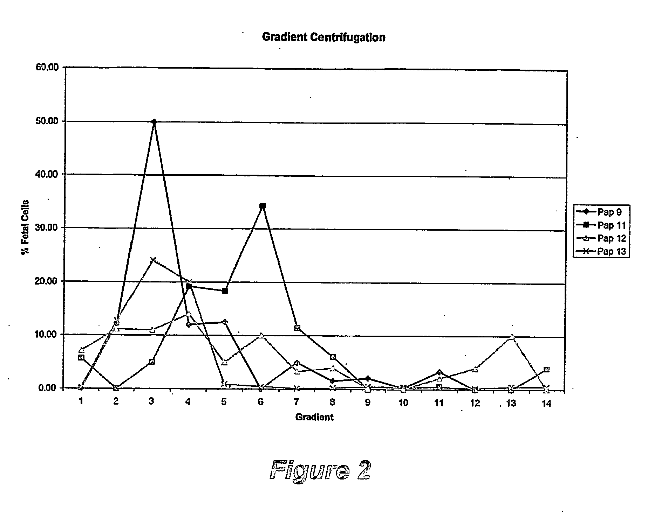

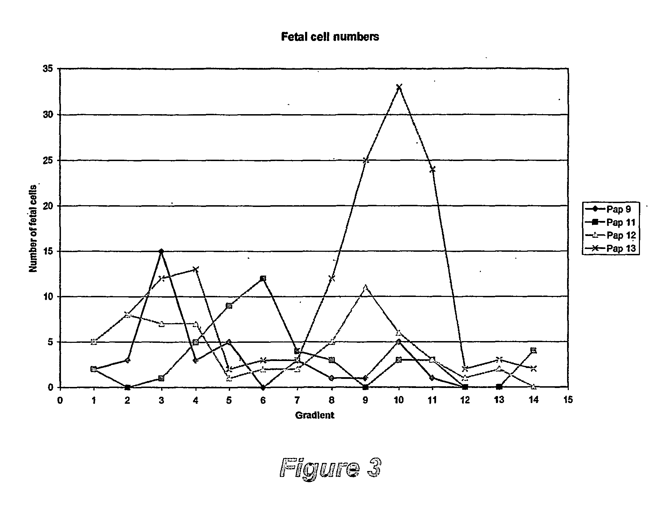

The percentage of fetal cells in each gradient, identified by double monoclonal antibody binding can be seen in Figure 2 whilst the total numbers of fetal cells in each gradient can be seen in Figure 3. These figures show that a high percentage of fetal cells are seen in gradients 2 to 7. The numbers of fetal cells in each gradient (Figure 3) show that there are two peaks containing fetal cells. The first peak corresponds with the peak in percentage of fetal cells seen in Figure 2. The second peak in fluorescent cells from gradients 8 to 11 were noted to be commonly attached to cell clumps that did not contain majority of fluorescent cells.

To improve the accuracy, reliability and cost effectiveness of non-invasive prenatal genetic diagnosis from pap smears, it is necessary to develop enrichment strategies that both reduce the concentration of contaminating cells and secondly recover as many fetal cells as possible. In previous prior art density gradient enrichment has been successfully used to enrich fetal cells contained within maternal blood samples. However the possibility of applying gradient enrichment to pap smears has not been thoroughly investigated with any degree of success. In this embodiment the percoll density gradient centrifugation method has been used to enrich fetal cells from pap smears.

This embodiment demonstrates gradient density centrifugation is easy to perform and can be used to enrich fetal cells 30 to 100 fold from that in the original sample. Using this method it was possible to identify gradients with 12-50% fluorescent cells, It was also observed that positive fluorescent cells in the maternal band (1.018-1.033g/mL) were commonly attached to cell clumps that contained mostly non-fluorescent cells. The use of digesting enzymes such as trypsin or collagenase prior to gradient centrifugation can be used release these cells, allowing them to travel through the gradient to their actual density, increasing the number of fetal cells retrieved.

In this embodiment prenatal diagnosis using fetal cells isolated from pap smears preferably requires a number of serial enrichment strategies in order to provide a reliable source of relatively uncontaminated fetal cells. Initial enrichment strategies usually identify cells using physical characteristics such as density, charge or size. Although not highly specific they do reduce target cell loss and are relatively low in cost. Secondary enrichment strategies often identify cells using specific cellular traits. These often require expensive reagents and therefore it is preferable to have samples with fewer cells that have been enriched using less costly methods, so that the amount of reagent consumed is minimized. Primary and secondary enrichment strategies must work in unison to provide a reliable source of uncontaminated fetal cells yet achieve maximum yield. Considering this, it may be preferable to collect cells from not just the most highly enriched density but also surrounding densities (excluding the enriched maternal band) such that the maximum yield of fetal cells is available for secondary enrichment, even though the percentage of fetal cells in the retrieved sample would be reduced.

In this example, we have shown that simultaneous percoll density gradient centrifugation can be used to consistently enrich fetal cells from pap smears 30 to 100 fold from that in the original cervical sample.

### EXAMPLE 2

### Isolation of fetal cells from PAP smears using four monoclonal antibody double positive selection with confirmation of cell origin by multiple fluorescent PCR

Pap smear samples and buccal swab samples were obtained from thirty-two pregnant women between 7 and 31 weeks gestation as using procedures described in Example 1.

An appropriate amount of cell suspension (2 to 60uL), based on cell concentration, was diluted to 60uL in PBS and spread on superfrost plus microscope slides (Menzel Glaser, Beunos Aires, Argentina) by gentle rotation. The slides were allowed to dry at room temperature overnight. Slides were then placed through the following protocol:
- 10 minutes in -20°C Methanol (BDH, Dorset, England) then allowed to air dry.
- Rehydrated 2 X 2mins in PBS
- 10 minutes 10% Donkey serum (Sigma-Aldrich, St. Louis, USA) diluted in PBS
- Donkey serum was removed
- 100uL of each of the following Primary Antibodies, 1/100 dilution in 10% donkey serum (*i*.*e*. final dilution 1/200) was then added.
   ab7816 Rabbit anti-human chorionic gonadotrophin antibody (Abcam, Cambridge, UK) and NCL-PLAP Mouse anti- placental alkaline phosphatase antibody (Novocastra Laboratories, Newcastle, UK).
- Slides were then incubate 90 minutes at room temperature in humidified chamber
- Slides were then washed 3 X 5 minutes in PBS
- 100uL of each following Secondary antibody 1/200 dilution in PBS (ie final dilution 1 /400) was then added.
   Donkey FITC polyclonal to rabbit IgG (Abcam, Cambridge, UK) and Chicken Rhodamine polyclonal to mouse IgG (Abcam, Cambridge, UK).
- Slides were then incubated 45 minutes at room temperature in humidified chamber
- Slides were then washed 2 X 5 minutes PBS
- Slides were then mounted in PBS with no coverslip.
- Slides were then scanned under a fluorescent microscope (Nikon, Melville, USA) with FITC and Rhodamine excitation filters. Total numbers of cells on the slide and the number and fluorescence levels of positive fluorescent cells were recorded. A representative sample of fluorescent cells from each patient was isolated by micromanipulation for MFPCR analysis. Cells were isolated using a heat elongated glass Pasteur pipette, with each isolate placed into a 0.2mL DNase and RNase free PCR tube (TreffLab, Degersheim, Gremany). Micromanipulation of the slide resulted in the fluorescent cell along with cells in the immediate proximity being isolated. Due to the concentration required to provide a significant population for immunohistochemistry analysis as well as cell clumping, isolation of single cells was not possible.

Isolated cells were then lysed by adding 1uL lysis buffer (200mM potassium hydroxide/50mM Dithiothretol), incubated at 65°C for 10 minutes then add 1uL neutralising buffer (300mM KCl/900mM Tris-HCl pH8.3/200mM HCl) [Cui, 1989]. Cells were stored at -80°C until MFPCR.

Multiplex fluorescent PCR of Amelogenin and STRs, D3S1358, Doss818, D7S820 and CSFIPO was performed on isolated cells. Each reaction contained forward and reverse primers, 1 X PCR buffer (Applied Biosystems, USA), 1.5mM MgCl₂ (Applied Biosystems, USA), 1.25mM each dNTP (Gibco, Life Technologies, Melbourne, Australia) and 1 unit Accuprime Taq (Invitrogen, Melbourne, Australia). PCR conditions were 94°C/2 minute denaturation followed by 45 cycles of 94°C/10 second denaturation, 57°C/l minute annealing and 68°C/30 second extension.

PCR product was processed using Ammonium acetate/Ethanol Clean-up. Post clean-up processing involved adding 2uL of cleaned-up product to 3uL loading buffer (Amersham Biosciences, Piscataway, New Jersey). Samples were then heated to 90 degrees for 60 seconds and placed immediately on ice. Analysis was completed using the Megabace 1000 capillary electrophoresis system with Genetic Profiler Version 1.5 software (Amersham Biosciences, Piscataway, New Jersey). Injection parameters were -3kV for 45 seconds and run parameters were -10kV for 75 minutes at 44°C.

The procedure to identify a fetal signal within that produced by MFPCR of the isolated cells was that outlined in Clayton et al., 1998., Forensic Science International 91 55-70. That is the STR is an additional band to that found in the maternal fingerprint i.e. consistent with maternal signal. It is not consistent with a stutter band or artefact peak and that it is the same base pair size as bands identified as fetal for the same locus within other isolations from the same patient.

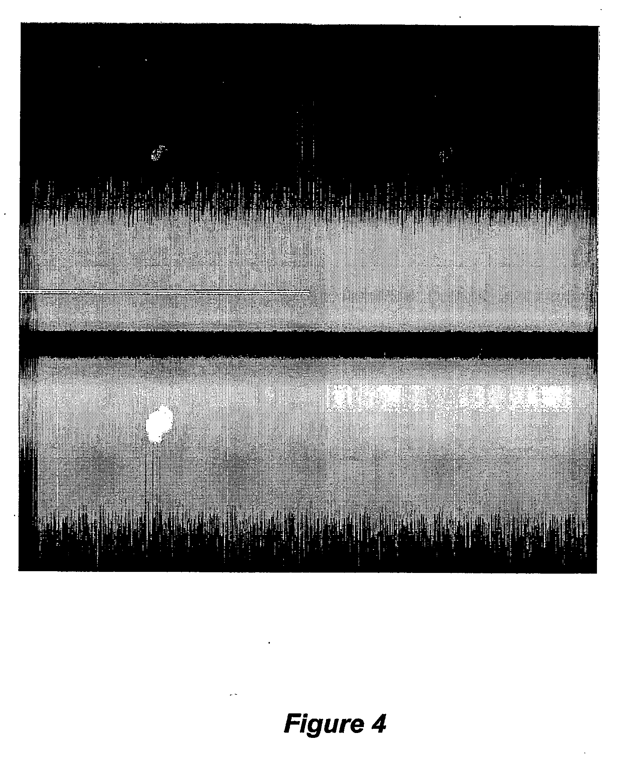

Representative photographs of high level and low level fluorescent cells for both FITC and Rhodamine are shown in Figure 4. This figure shows relative fluorescence levels compared to background (surrounding) cells as well as the typical morphology of the isolated cells.

Table 2 is an overview of the results obtained with both immunohistochemistry and MFPCR. Within this table is the gestation of each patient, the percentage of cells with a high level of fluorescence (++) and the overall percentage of cells exhibiting fluorescence higher than the background for each antibody set. Also shown is the number of high level fluorescent cells isolated, the total number of cells isolated with fluorescence higher than background levels, the percentage of both these cell sets that exhibited a fetal signal. The number of cells on the slide has been extrapolated to indicate the number of cells obtained from the sample. From the number of cells in the sample, the percentage with positive fluorescence, and the percentage exhibiting a fetal DNA fingerprint, the number of fetal cells within the sample can easily be determined. Table 2 shows that all patients showed fluorescent cells for both antibody sets. Fetal cells were also detected by MFPCR on at least one cell from the majority of patients.

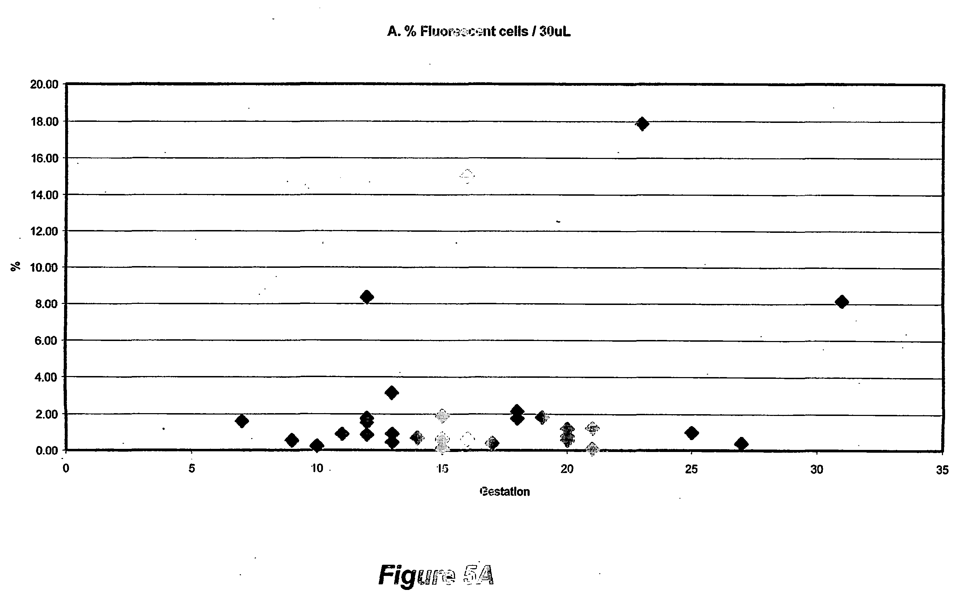

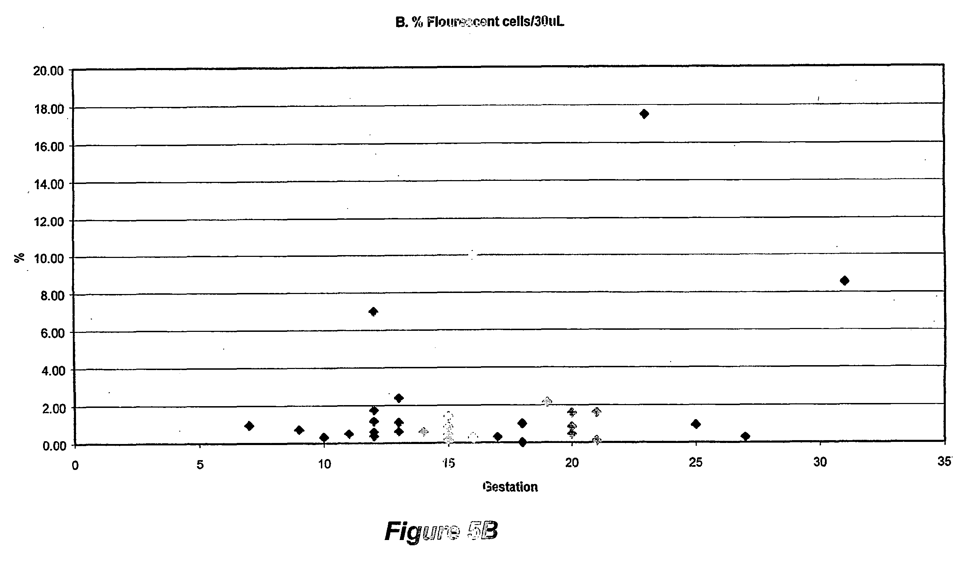

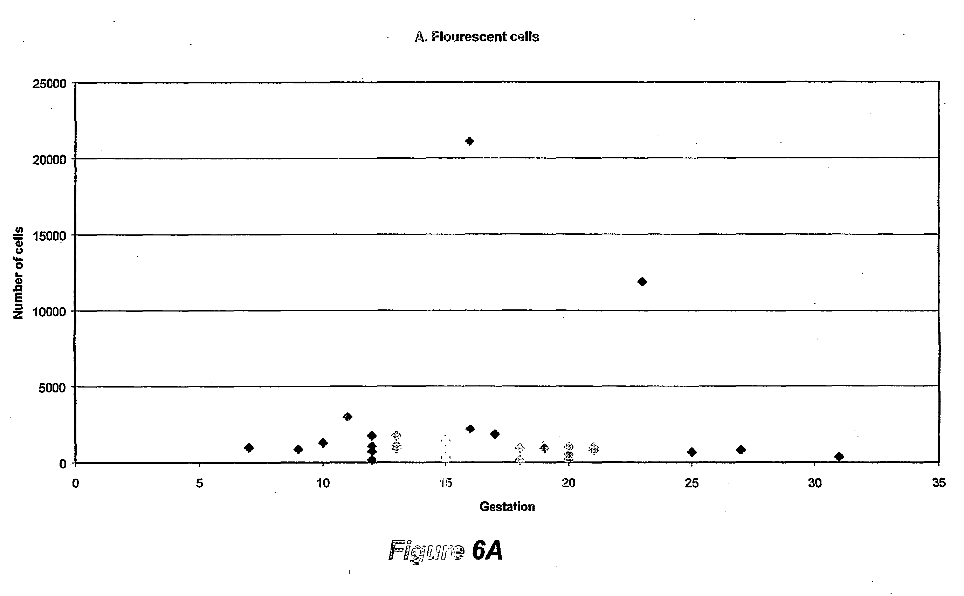

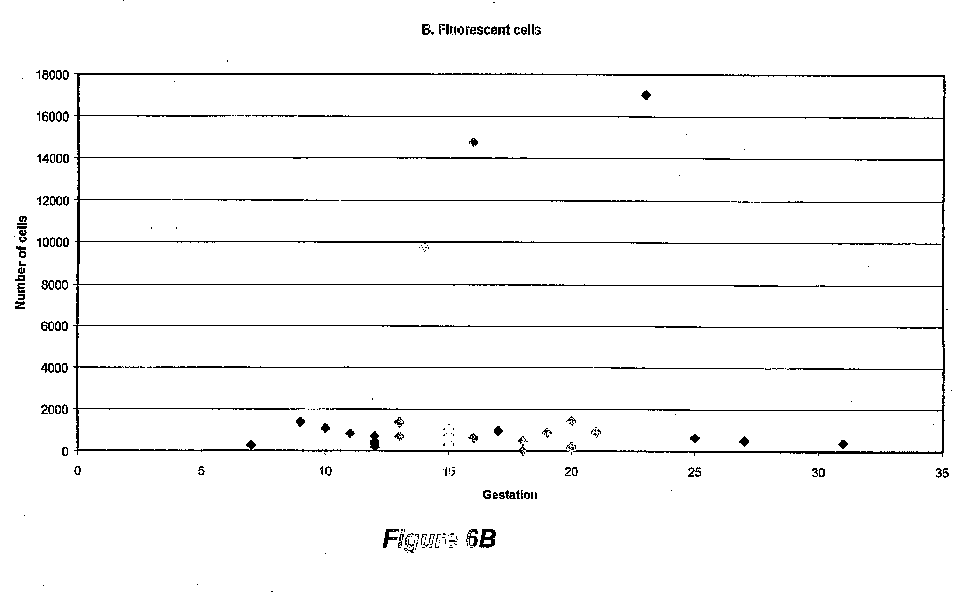

The percentage of fluorescent cells with reference to the gestation of the pregnancy can be seen in Figure 5A and 5B. Figure 5A shows antibodies to human placental lactogen (PLac) and folate binding protein (LK26), whilst Figure 5B shows antibodies to HCG and PLAP. Both of these graphs indicate that there is no apparent relationship between the percentage of fluorescent cells and the gestation of the pregnancy. The total numbers of fetal cells in the sample is compared to the gestation of the pregnancy in Figure 6A and 6B. Figure 6A shows antibodies to PLac and LK26, whilst Figure 6B shows antibodies to HCG and PLAP. Both of these graphs show that there appears to be no correlation between the number of fluorescent cells in the sample and the gestation of the pregnancy. Figures 5 and 6 both show that cells with positive fluorescence for both antibody sets were exhibited in all samples.

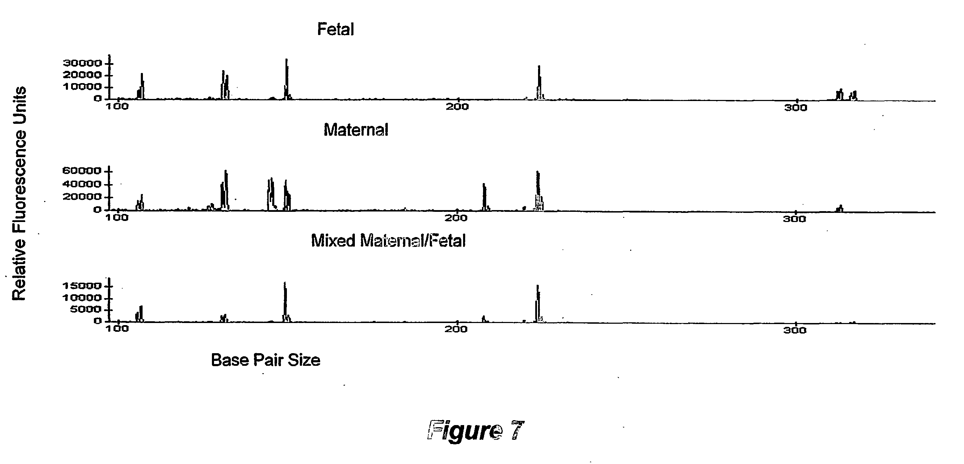

A representative electrophoretic profile of the multiplex from maternal, fetal and mixed maternal/fetal samples analysed by capillary electrophoresis is shown in Figure 7. These profiles show that the fetal signal is consistent with being the progeny of the mother, with at least 1 allele the same base pair size for each locus. The mixed sample shows a maximum of 3 alleles at each locus with two being consistent with the maternal signal and the third assumed to be the paternally derived allele.

The level of specificity confirmed by MFPCR for each set of antibodies towards fetal cells is shown in Table 3. This shows that a higher specificity is obtained with Antibody set A (PLac and LK26). Table 2 also shows that the specificity observed with cells exhibiting low level fluorescence is similar to that with high level fluorescence.

Enrichment and diagnosis of fetal cells from the cervix must be consistently successful if it is to become an alternative to invasive procedures such as amniocentesis or chorionic villus sampling. This invention demonstrates that prenatal genetic diagnosis using minimally invasive procedures is, for the first time, a very real prospect. However alternatively it should also be appreciated that this invention may also be considered as a complementary technique to other non-invasive or minimally invasive tests such as biochemical screening and ultrasound screening offered to pregnant women during the first trimester of pregnancy (Daryani et al., 2000, J. Obstet. Gynecol. 183 752).

Efficient monoclonal antibody mediated detection of fetal cells currently requires a combination of highly specific antibodies. In this invention the specificity of two sets of double positive monoclonal antibodies for selection of fetal cells was used. The results indicate that cells reacting against both sets of antibodies are present in all 32 patients. The percentage of fluorescent cells range from 0.08 to 15.76%, with no apparent correlation to gestation. The overall number of fluorescent cells in the sample also varies with no correlation to gestation. This may indicate that variation in the number of fetal cells in the sample is specific to the patient or perhaps more likely due to variations in the technique used by the operator performing the retrieval.

DNA fingerprinting using MFPCR was used to confirm cell origin of the fluorescent cells from each antibody set and patient. This MFPCR technique has the advantage of being highly discriminating for cell origin even when applied to very close relatives such as mother and baby. A small number of representative fluorescent cells were isolated by micromanipulation from the slide after immunohistochemical analysis. Due to the concentration required to provide a significant population for immunohistochemistry analysis as well as cell clumping, isolation of single cells was not possible. Therefore the percentages of fetal cells in the sample should be considered a minimum possible value. Also due to the relatively small numbers of cells tested on each patient the number of fetal cells in the sample, extrapolated from the percentage of fetal profiles are to be considered approximate values.

Combining the antibody and MFPCR data from all patients, it is possible to determine a minimum specificity for each antibody set towards fetal cells. Table 3 indicates that the specificity for antibody set A (PLac and LK26) is higher than that for antibody set B (HCG and PLAP). It can also be seen that specificity for low level fluorescence is similar to that for high level fluorescence. Although these specificities, from 0.40 to 0.54, appear quite low considering two highly specific antibodies towards fetal antigens bound to the cell, this may be due to non-specific binding to non-cellular particles, binding to non-intact cells or strong binding to a maternal cell lineage (less likely). The former two more likely options would cause a maternal profile due to surrounding cells and therefore artificially reduce the specificities of each antibody set. However false positive results or identifications would nevertheless not be obtained as there would be no template present to facilitate amplification of fetal alleles.

Genetic diagnosis of limited numbers of fetal cells obtained from the uterine cervix using techniques such as FISH and PCR is very limited. FISH analysis can only identify fetal cells if they are aneuploid or originate from a male fetus (Fejgin *et al*., 2001, *supra*) - this is an important and considerable limitation to the use of such techniques for prenatal diagnosis. Other studies use PCR analysis to detect disorders however in most cases this is limited to the gene analysed and quantitative variations in the maternal and fetal alleles, for example RH(D) analysis (Tutschek et al., 1995, Prenatal Diagnosis 15 951). Again this is an important and considerable limitation to the use of such techniques for prenatal diagnosis.

However MFPCR has the advantages of overcoming these limitations, as it is not limited by sex or individual gene alleles. MFPCR has an extremely high level of discrimination between closely related individuals, can be performed on single cells and provides multiple diagnoses within a single reaction.

In this embodiment, MFPCR was used top accurately determine the presence of fetal cells in a mixed fetal/maternal sample. For these reasons we suggest that MFPCR be considered the preferred method of choice when performing prenatal genetic diagnosis from pap smear samples.

MFPCR analysis of limited numbers of isolated fluorescent cells from each patient identified a fetal profile in 28 out of 32 samples. Due to the relatively small numbers of analysed cells on each patient and highly specific selection criteria, this is not indicative of fetal cells being absent in the four patients without a fetal signal.

### EXAMPLE 3

### Serial enrichment of fetal cells from PAP smears by gradient centrifugation and MACS: Identification by immunohistochemistry and confirmation by multiplex fluorescent PCR DNA fingerprinting

Ten pap smear samples and buccal swab samples were obtained from pregnant women between 7 and 31 weeks gestation using procedures described in Example 1.

### Step 1: Trypsin Digestion

- Split each sample into 3 X 200uL + 1 X 50uL - 200uL (or 50uL) 2.5% Gibco Trypsin/EDTA in PBS was added
- Incubated 37 degrees for 2 hours
- 1mL PBS was added and then centrifuged 3000rpm 5 minutes (Biofuge pico)
- Supernatant was removed
   50uL initial material sample was placed in -20°C freezer for later analysis

### Step 2: Gradient centrifugation

A 2:1 dilution of percoll (Amersham Biosciences, Piscataway, New Jersey) in PBS was prepared and 3mL of percoll/PBS was added to a Quick-Seal centrifugation tube (Beckman Coulter, Fullerton, CEA). 30uL pap smear cell suspension was layered onto the pereoll/PBS and centrifuged at 30000rpm (22000g) in Beckmann ultrafuge (Beckman Coulter, Fullerton, CA) for 30 minutes at room temperature using vacuum. Isolation of gradients

Break 22 gauge syringe needle (Terumo Medical Corporation, Elkton, MD) neatly and pierce bottom of centrifuge tube leaving syringe embedded. Allow to drain into 15 mL Falcon tubes (BD Biosciences Discovery Labware, Bedford, MA). Drain to waste first 50 uL, collect next 1800 uL (or until maternal band touches top of syringe).

Use empty syringe to apply pressure if needle becomes blocked.

Add 13mL PBS to each tube end centrifuge 2000RPM 5 minutes (Sigma 4K15 centrifuge).

Draw off supernatant and transfer cells to 1.5mL centrifuge tube and wash with 800uL PBS to ensure all cells are transferred. Spin 3000rpm (microfuge) 5 min and remove supernatant.

Place 1 tube from each patient in -20 freezer for analysis.

Place specimens in fridge overnight.

### Step 3: Magnetic Bead Isolation (1^{st} pass)

### A. Preparation of Dynabeads M-280 Streptavidin

The Dynabeads should be washed before use to remove the 0.02% NaN₃ added as a preservative. The washing procedure is facilitated by using a magnet (Dynal MPC).
1. Resuspend the Dynabeads by gently shaking the vial to obtain a homogeneous suspension.
2. Add 50uL of Dynabeads to a 1.53mL centrifuge tube.
3. Add 400uL 2XBinding Buffer (10mM Tris-HCl pH 7.5, ImM EDTA, 2M NaCl) to tube
4. Place the tube on the magnet for 1-2 min. Do not remove the tube from the magnet during the separation process.
5. Remove the supernatant by aspiration with a pipette while the tube remains on the magnet. Avoid touching the inside wall of the tube, where the Dynabeads are attracted to the magnet with the pipette tip.
6. Remove the tube from the magnet. Add 400uL 2XBinding buffer along the inside of the tube where the Dynabeads are collected.
7. Repeat steps 3 to 5 three times and after the last wash add 60uL 1Xbinding buffer to obtain an appropriate working concentration of Dynabeads.

### II. Secondary Antibodies.

1. Add 10uL (10mg/mL) of Donkey anti-rabbit secondary antibody to 60uL prepared Dynabeads.
2. Incubate at room temperature for 120 minutes with gentle rotation of the tube and occasional vortex.
3. Separate the Dynabeads now coated with biotinylated antibodies using a magnet for 1-2 minutes.
4. Wash 4 times with 400uL PBS using the magnet.
5. Resuspend in 200uL PBS/DS

### Indirect technique:

1. Add 400uL PBS/DS to each sample tube, vortex then incubate et room temperature for 10 minutes.
2. Centrifuge 3000rpm (microfuge) for 5 minutes then draw off supernatant.
3. Add 30uL PBS/DS
4. Dilute 20uL anti-Plac with 200uL PBS
5. Add 10uL diluted anti-PLac to each sample tube and incubate for 150 min at room temperature with gentle rotation.
6. Add 1000uL PBS spin at 3000rpm 5 minutes (microfuge)
7. Repeat wash X 2 and resuspend in 20uL PBS/DS
8. Add 10uL secondary Ab coated Dynabeads^{®} to each sample tube and incubate for 90 minutes at room temperature with gentle rotation and intermittent vortex.
9. Capture rosetted cells by adding 400uL PBS then applying the tube to a Dynal MPC^{®} magnet 1-2 min draw off supernatant. Supernatant containing non-rosetted cells is removed whilst rosetted cells remain on the tube wall.
10. Remove the tube from the magnet and wash cells with 400uL PBS. Place tube back on magnet and repeat wash 2 times.
11. Resuspend in 30uL PBS.
12. Remove 1 sample from each specimen and place in -20 freezer for analysis
13. Place specimens in fridge overnight

### Step 4: Magnetic Bead Isolation (2^{nd} pass)

Prepare 30uL Dynabeads M-280 Streptavidin as described in step 3.

### A. Secondary Antibodies.

6. Add 10uL (10mg/mL) of Chicken anti-mouse secondary antibody to 60uL prepared Dynabeads.
7. Incubate at room temperature for 120 minutes with gentle rotation of the tube and occasional vortex.
8. Separate the Dynabeads now coated with biotinylated antibodies using a magnet for 1-2 minutes.
9. Wash 4 times in 400uL PBS using a magnet
10. Resuspend in 100uL PBS/DS

### Indirect technique

1. Add 100uL 2.5% Trypsin, to release cells from beads, to each tube and incubate at 37°C for 2 hours.
2. Add 1000uL PBS to each tube
3. Centrifuge at 3000rpm (microfuge) for 5 minutes then draw off supernatant
4. Repeat wash 2 more times
5. Add 30uL PBS/DS
6. Dilute 10uL anti-LK26 with 100uL PBS
7. Add 10uL diluted anti-PLac and incubate for 150 min at room temperature with intermittent mixing.
8. Add 1000uL PBS spin at 3000rpm 5 minutes (microfuge)
9. Repeat wash X 2 and resuspend in 20uL PBS/DS
10. Add 10uL secondary Ab coated Dynabeads^{®} to cells and incubate for 90 minutes at room temperature with gentle rotation and intermittent vortexing.
11. Capture rosetted cells by adding 200uL PBS then applying the tube to a Dynal MPC^{®} magnet 1-2 min draw off supernatant. Supernatant containing non-rosetted cells is removed whilst rosetted cells remain on the tube wall.
12. Remove the tube from the magnet and wash cells with 200uL PBS. Place tube back on magnet and repeat wash 2 times.
13. Resuspend in 30uL PBS.
Place samples in -20° C freezer for analysis.

### Immunohistochemistry

Trypsin digestion and density gradient centrifugation samples were analysed by double monoclonal antibody immunohistochemistry. Cell suspension was dropped onto superfrost plus microscope slides (Menzel Glaser, Beunos Aires, Argentina), tube was washed with 20uL PBS to ensure all cells are transferred onto slide and spread by gentle rotation. The slides were allowed to dry at room temperature overnight. Slides were then placed through the following protocol:
- 10 minutes at -20°C in Methanol (BDH, Dorset, England) then allow slide to air dry.
- Rehydrate 2 X 2mins in PBS
- 10 minutes in 10% Donkey serum (Sigma-Aldrich, St. Louis, USA) diluted in PBS
- Draw off donkey serum
- Add 100uL each Primary Antibody, 1/100 diluted in 10% donkey serum (ie final dilution 1/200)
   Rabbit anti- human placental lactogen antibody (Neomarkers, Fremont, CA, USA) and Mouse LK26 anti-trophoblast and ovarian cancer antibody (Signet Pathology Systems, Dedham, MA, USA).
- Incubate 90 minutes at room temperature in humidified chamber
- Wash 3 X 5 minutes in PBS
- Add 100uL each Secondary antibody 1/200 dilution in PBS (ie final dilution 1/400)
   Donkey FITC polyclonal to rabbit IgG (Abcam, Cambridge, UK) and Chicken Rhodamine polyclonal to mouse IgG (Abcam, Cambridge, UK).
- Incubate 45 minutes at room temperature in humidified chamber
- Wash 2 X 5 minutes in PBS
- Mount slides in PBS with no coverslip.

Slides were then scanned under a fluorescent microscope (Nikon, Melville, USA) with FITC and Rhodamine excitation filters. Total numbers of cells on the slide and the number of positive fluorescent cells and fluorescence levels were recorded.

### MFPCR

Antibody enriched samples were analysed using MFPCR. Single cells were visualized under an inverted microscope (Leica Microsystems, Wetzlar, Germany) with X40 magnification and isolated using a heat elongated glass Pasteur pipette into 0.2mL DNase and RNase free PCR tubes (TreffLab, Degersheim, Germany). Tubes were stored at -80°C until lysis.

Isolated cells were lysed by adding 1uL lysis buffer (200mM potassium hydroxide/50mM Dithiothreitol), incubated at 65°C for 10 minutes then add 1uL neutralising buffer (300mM KCl/900mM Tris-HCl pH8.3/200mM HCl). Cells were stored at -80°C until MFPCR.

Multiplex fluorescent PCR of Amelogenin and STRs D3S1358, D5S818, D7S820, CSF1P0, THO, FGA, D21S11, and D18S51 was performed on isolated cells using previously described protocols (Findlay *et al.,* 2001, *supra*; International Application PCT/AU02/01388). Each reaction contained forward and reverse primers, 1 X PCR buffer (Applied Biosystems, USA), 1.25mM each dNTP (Gibco, Life Technologies, Melbourne, Australia) and 1 unit Qiagen HotStarTaq (Qiagen Melbourne, Australia). PCR conditions were 94°C/2 minute denaturation followed by 45 cycles of 94°C/10 second denaturation, 57°C/1 minute annealing and 68°C/30 second extension.

PCR product was processed using Ammonium acetate/Ethanol Clean-up. Post clean-up processing involved adding 2uL of cleaned-up product to 3uL loading buffer (Amersham Biosciences, Piscataway, New Jersey). Samples were then heated to 90 degrees for 60 seconds and placed immediately on ice. Analysis was completed using the Megabace 1000 capillary electrophoresis system with Genetic Profiler Version 1.5 software (Amersham Biosciences, Piscataway, New Jersey). Injection parameters were -3kV for 45 seconds and run parameters were -10kV for 75 minutes at 44°C.

The procedure to identify a fetal signal within that produced from MFPCR of the isolated cells was that outlined in Clayton *et al.,* 1998, *supra.*

Table 4 shows then gestation of each patient that pap smears were taken from. Also shown is macroscopic examination of the sample observing the presence of blood, numbers of cells determined by how opaque the sample was and the presence of large cell clumps.

The percentage of fetal cells in the original sample was identified by immunohistochemistry (Table 5). Positive cells were those that exhibited both FITC and rhodamine fluorescence. After enrichment by density gradient centrifugation, the percentage of fetal cells in the retrieved sample was also determined by immunohistochemistry, allowing calculation of the fold enrichment and the percentage loss of fetal cells by this technique (Table 5). Following enrichment using antibodies to placental lactogen (1^{st} pass) and LK26 (2^{nd} pass) the retrieved cells were micromanipulated and cell origin was determined by MFPCR. MFPCR of these cells showed either maternal cells, with alleles the same as the maternal fingerprint, or fetal cells which were consistent with being the progeny of the mother. A third set of cells were also present which could not be classed as either maternal or fetal because of a lack of information due to allele dropout, primer or PCR failure. These inconclusive cells were designated as maternal cells so that the relative number of fetal cells would not be artificially heightened. Therefore the results should be considered a minimum level of enrichment. Using the MFPCR analysis the percentage of fetal cells, fold enrichment and the level of fetal cell loss compared to the unprocessed sample could be determined (Table 5).

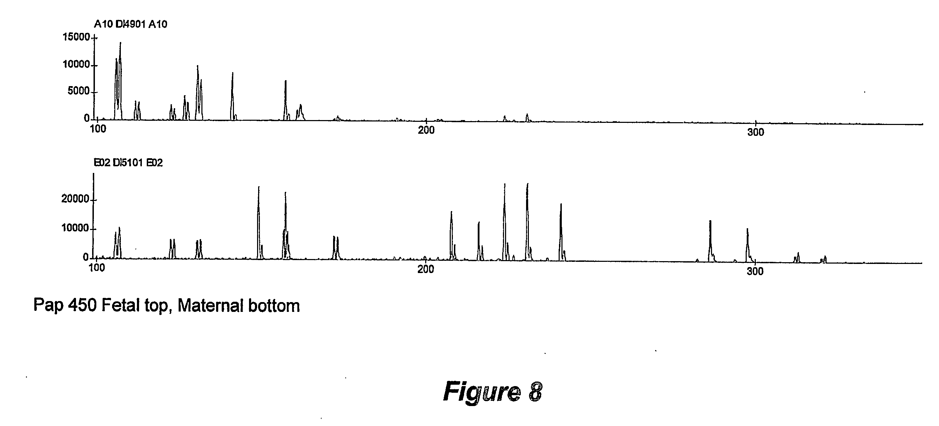

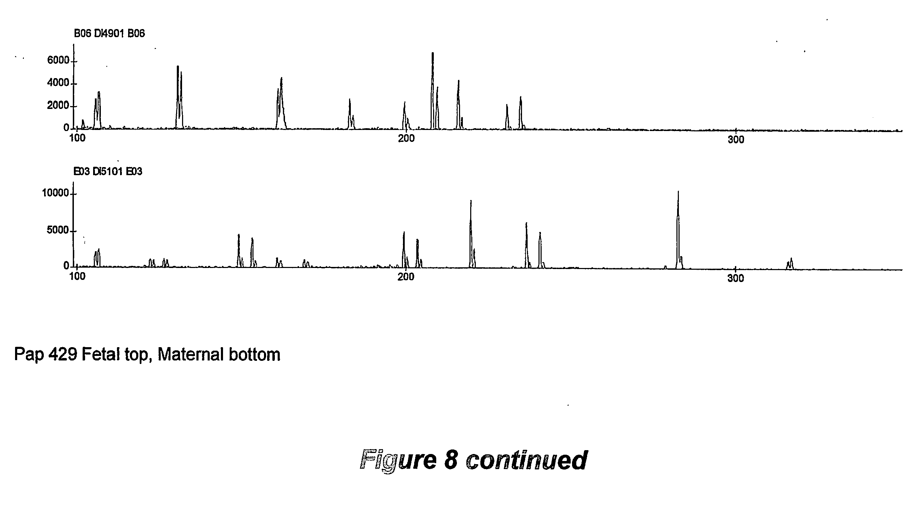

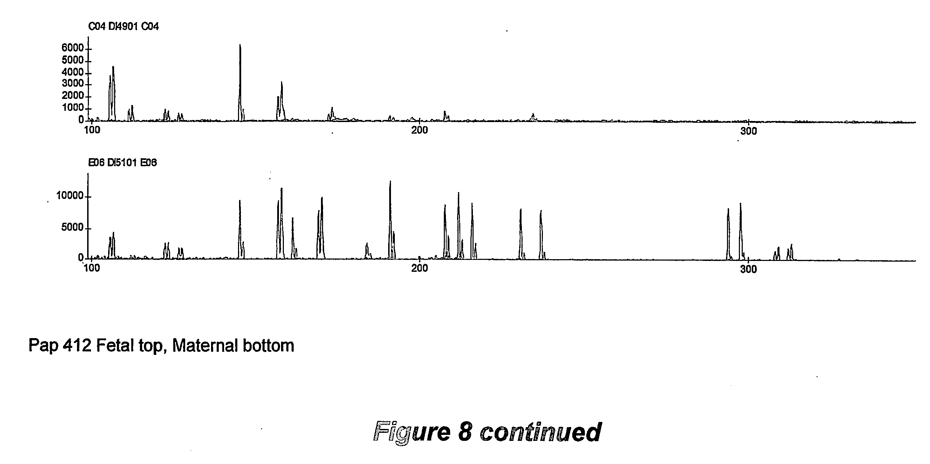

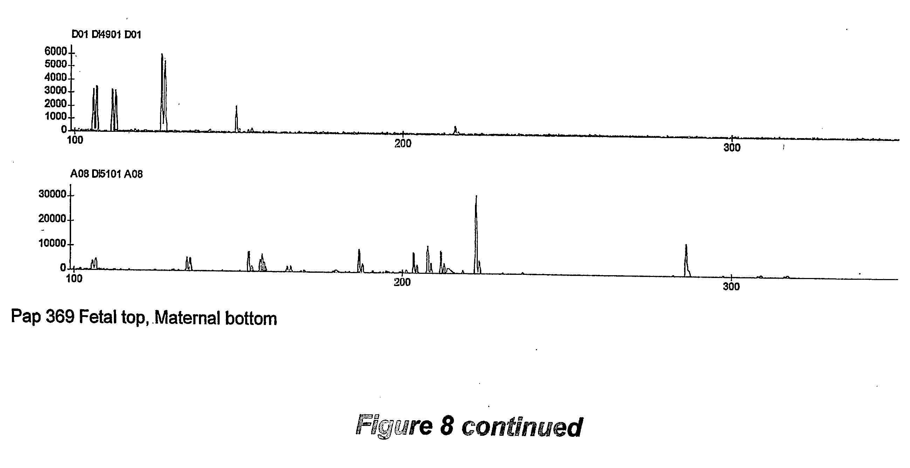

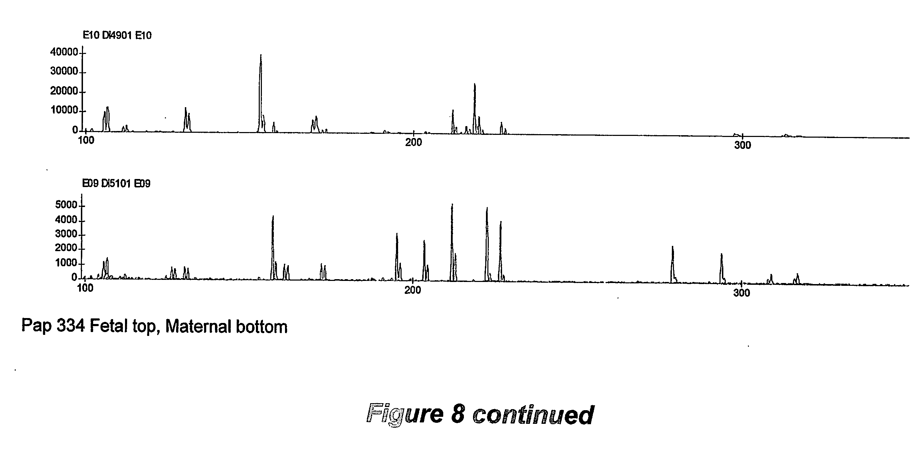

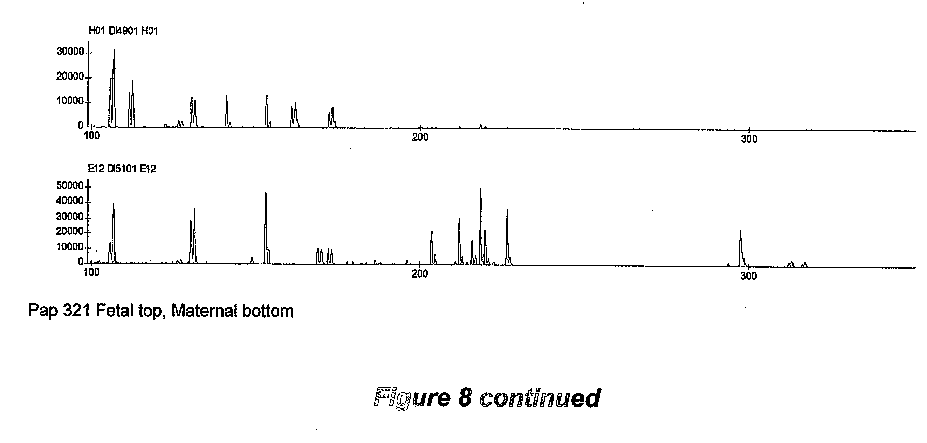

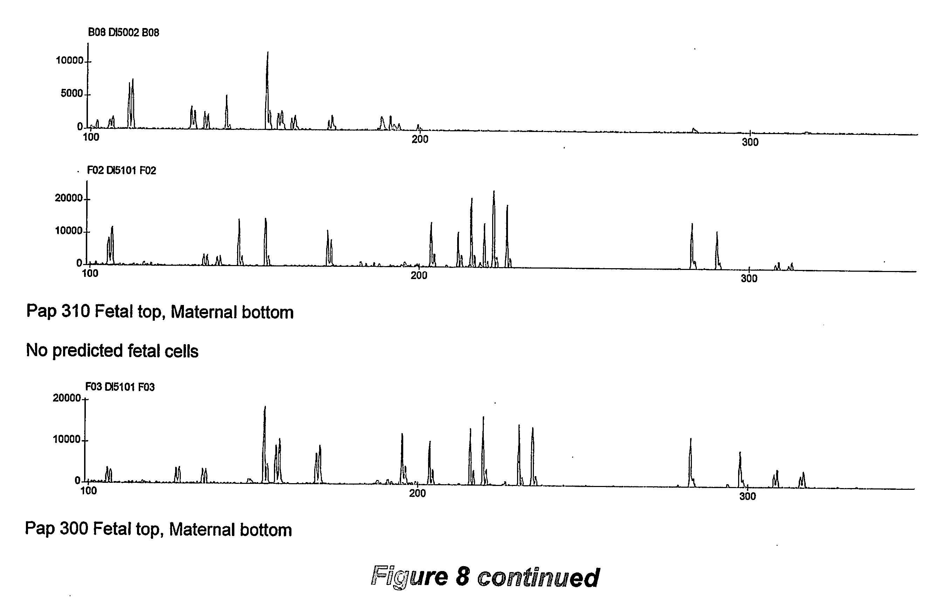

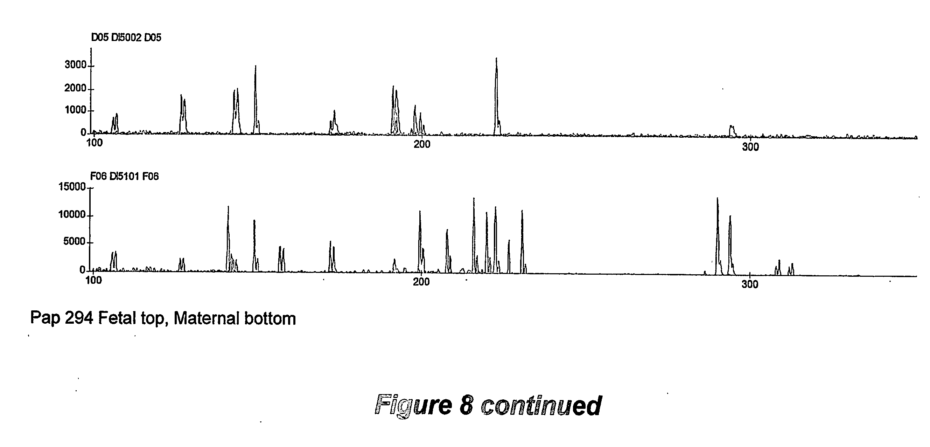

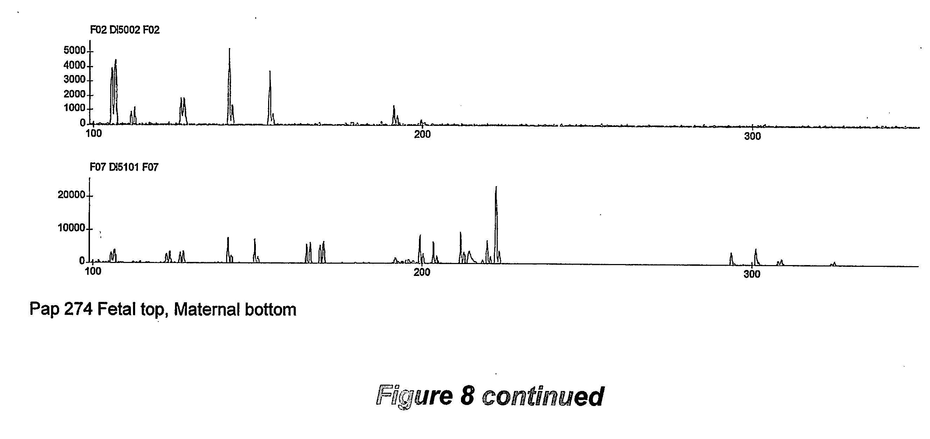

Figure 8 contains representative electropherograms of maternal cells and isolated fetal cells. These electropherograms show that the fetal signal is consistent with being the progeny of the mother. A significant level of allele dropout and primer failure can also be seen in the fetal fingerprints, causing the group of cells that could not be identified conclusively as either maternal or fetal. This allele dropout and primer failure was not observed in the cell suspension or single buccal cell positive controls on each plate.

This embodiment also indicates that cells of fetal origin are indeed present in the endocervical canal of the mother. Due to the relatively low number of fetal cells present serial enrichment strategies must be utilized to in unison to provide a reliable source of uncontaminated fetal cells yet still provide maximum yield. Once a reliable source of fetal cells is established, the diagnostic techniques such as MFPCR used to screen these cells needs to not only confirm fetal origin and but also test for genetic traits.

Prior to enrichment the sample was analyzed macroscopically for the presence of blood, numbers of cells determined by how opaque the sample was and the presence of large cell clumps. The sample was then treated with trypsin to dissociate cells from cervical mucus and clumps. Immunohistochemistry was performed on of the sample after trypsin digestion to determine the number of cells presenting target antigens (fetal cells) in the original sample. The numbers of fetal cells varied from 0.1 to 3.7 percent with no correlation to gestation. Microscopic evaluation of the number of cell clumps was also performed confirming that digestion had released the majority of cells from clumps present in the original sample.

Again primary and secondary enrichment strategies must work in unison to provide both a reliable source of uncontaminated fetal cells with maximum yield. Therefore in this study, cells were collected from not just the most highly enriched density but also surrounding densities (1.049-1.142g/mL) such that the maximum yield of fetal cells was available for secondary enrichment, even though the percentage of fetal cells in the retrieved sample was reduced. The level of enrichment by gradient centrifugation varied between patients from 2 to 17 fold with a mean of 10 fold. Furthermore the percentage of fetal cells in the enriched sample, identified by double monoclonal antibody immunohistochemistry varied from 2 to 17 percent Confirmation of centrifugation can be easily performed by macroscopic visualization of the maternal band between 1.018-1.033g/mL.

In this embodiment antibodies to human placental lactogen and folate binding protein (LK26) were applied to the MACS system to enrich for cells exhibiting target antigens. Using this technique the percentage of fetal cells in the retrieved sample, identified by MFPCR, were significantly increased from that in the gradient centrifugation enriched sample previously outlined. Each MACS enrichment step appears to enrich the number of fetal cells from that in the previous sample. Although the numbers of cells available for analysis after MACS enrichment was quite low (2-40 cells) due to the specimen being split and only 25% of the sample used to analyze each stage of enrichment, fetal cells were identified in 9/10 of samples whilst the levels of fetal cells in the sample varied from 17-70 percent, representing an average of 117 fold enrichment in the final sample when compared to the original sample. Although both fluorescent activated cell sorting (FACS) and MACS can both be used to enrich for fetal cells from pap smears, MACS is far less expensive and easier to perform than FAGS. MACS is a fast, easy to handle, inexpensive technique which can easily be adapted to a high throughput platform.

MFPCR was used to confirm cell origin of single cells isolated using MACS. MFPCR of these cells showed either maternal cells, with alleles the same as that of the mother or fetal cells which were consistent with being the progeny of the mother. A third set of cells were also present which could not be classed as either maternal or fetal because of a lack of information due to allele dropout, primer or PCR failure. Positive controls such as the cell suspension or single buccal cells run in parallel did not exhibit this level of allele dropout or primer failure. This indicated that the reduced number of peaks from the isolated fetal cells may be due to their significant processing and may be less susceptible to cell lysis and histone denaturation using the alkaline method described.

Again MFPCR has the advantage of being highly discriminating for cell origin even when applied to close relatives such as mother and baby. MFPCR can be performed on single cells and provides multiple diagnosis within a single reaction. In this embodiment MFPCR was used to accurately determine the presence of fetal cells isolated from a mixed fetal/maternal sample. This embodiment demonstrates that samples highly enriched in fetal cells can be produced even though an uncontaminated source of fetal cells from pap smears (i.e. isolation of 100% fetal cells) may not be possible. Single fetal cells can then be easily isolated and used to screen for genetic traits. For this reason we suggest that MFPCR be considered the method of choice when performing prenatal genetic diagnosis from pap smear samples.

### EXAMPLE 4

### Optimization of MACS enrichment of fetal cells

A preferred embodiment of MACS isolation requires two sequential positive selection steps using antibodies directed to fetal antigens such as human placental lactogen and folate binding protein. The antibody to human placental lactogen is a rabbit antibody, which requires a secondary, biotinylated donkey anti-rabbit antibody for streptavidin labeled Dynabead binding. The antibody to folate binding protein is a mouse antibody which requires a secondary, biotinylated chicken anti-mouse antibody for Dynabead binding.

MACS enrichment efficiency can be compromised by secondary antibody binding non-specifically to maternal cells.

To address this potential problem, the present inventors have optimized MACS enrichment by first incubating the post-density enriched cell population with non-biotinylated secondary antibody prior to incubation with each of the antibodies to human placental lactogen and folate binding protein according to the following procedure.

### Cell preparation and density gradient enrichment

Pap smear cells were taken from storage at -80°C and thawed at room temperature while noting sample ID, cell number and presence of blood e.g. 755 cells = +++, blood = ++

To 150 uL of cell suspension was added 150 uL of trypsin followed by incubation in an oven at 37°C for 2 hours, vortexing briefly approx every 30 minutes.
800ul of PBS was then added to the trypsinized cells which were then centrifuged at 3000rpm for 5 minutes (Pico microfuge). The trypsin supernatant was then removed leaving 100uL of cell suspension in tube.

A continuous, simultaneous Percoll density gradient was formed using 3ml of 2:1 percoll:PBS in 12 Quick-Seal centrifugation tube (Beckman Coulter, Fullerton, CA) with the following procedure.
- Layer 100u1 of the pelleted cells to the top of the gradient medium
- Wash the tube with 20ul of PBS and add this to the gradient medium
- Spin samples at 30,000 rpm for 30 minutes under vacuum (Beckmann ultrafuge Beckman Coulter, Fullerton, CA)
- Break 22 gauge syringe needle (Terumo Medical Corporation, Elkton, MD) neatly and pierce bottom of centrifuge tube leaving syringe embedded
- Allow to drain into 15 mL Falcon tubes (BD Biosciences Discovery Labware, Bedford, MA). Drain to waste first 50 uL (6-8 drops), collect next 1800 uL, or until maternal band touches top of syringe.
- Use empty syringe to apply pressure if needle becomes blocked.
- Add 13 mL PBS to each falcon tube (this dilutes out the percoll)
- Spin the sample at 1500rpm for 10minutes ((Sigma 4K15 centrifuge)
- Remove supernatant leaving 500ul and transfer to a 1.5ml eppendorf tube
- Wash falcon tube with 2x500ul of PBS and transfer to eppendorf tube to ensure all cells are transferred.
- Spin samples at 3000rpm for 5 minutes (Pico microfuge)
- Remove supernatant leaving 100ul of cell suspension
- Store samples in -4°C fridge overnight.

### MACS Enrichment (Based on 6 samples)

### 1^{st} Secondary antibody blocking step

- Dilute 3 uL non-biotinylated polyclonal donkey anti-rabbit antibody in 120 uL donkey serum
- Incubate at room temperature for 10 minutes
- Add 1mL of PBS to each tube to wash the sample
- Spin samples at 3000rpm for 5 minutes (Pico microfuge)
- Remove 1mL of supernatant leaving 100u1 in tube
- Repeat above wash steps a further 2x
- (Cells should be left in 100ul of PBS)
- Add 6 uL anti-placental lactogen antibody to 60uL donkey serum
- Add 10 uL anti-placental lactogen antibody/donkey serum to each sample
- Incubate tubes at room temperature for one hour on a shaker

### Dynabeads M-280 Streptavidin Preparation

- The Dynabeads should be washed before use to remove the 0.02% NaN₃ added as a preservative.
- Resuspend the Dynabeads by gently shaking the vial to obtain a homogeneous suspension
- Add 30 uL (5uL per sample) of Dynabeads to a 1.5 mL centrifuge tube.
- Add 200 uL 2x Binding Buffer (10mM Tric-HCl pH 7.5, 1mM EDTA, 2M NaCl on the bench) to the tube
- Place the tube on the magnet for 1-2 min without removing the tube from the magnet during the separation process.
- Remove the supernatant by aspiration with a pipette while the tube remains on the magnet.
- Remove the tube from the magnet and add 200 uL 2x binding buffer along the inside of the tube where the Dynabeads are collected.
- Repeat above wash steps a further 3 times and at the last wash resuspend the sample in 30 uL of 1x binding buffer (i.e. add 15 uL of 2x binding buffer and 15 uL of MilliQ H₂0 to make the binding buffer 1x)
- Add 6 uL (1uL per sample) of biotinylated polyclonal donkey anti-rabbit antibody to the magnetic beads, vortex briefly
- Incubate tubes at room temperature for one hour on a shaker

### Washing samples

- Add 1ml of PBS to each tube to wash the sample
- Spin samples at 3000rpm for 5 minutes (Pico microfuge)
- Remove 1mL of supernatant leaving 100 uL in tube
- Repeat above wash steps a further 2 times
(Cells should be left in 100 uL of PBS)

### Washing Dynabeads

- Wash the Dynabeads while the samples above are spinning after their 2^{nd} wash
- Add 200 uL of PBS to the Dynabeads
- Place the tube on the magnet for 1-2 minutes, do not remove the tube from the magnet during the separation process
- Remove the supernatant by aspiration with a pipette while the tube remains on the magnet.
- Resuspend the beads in 200 uL of PBS
- Repeat above wash steps a further 3 times
- Resuspend the magnetic Dynabeads in 60uL (10uL for each sample) of donkey Serum
- Add 10uL of Dynabeads above to each sample and vortex briefly
- Incubate tubes at room temperature for one hour on a shaker

### Washing samples

- Take samples off the shaker, vortex briefly and add 300 uL of PBS to the samples
- Place the tube on the magnet for 1-2 minutes without removong the tube from the magnet during the separation process
- Remove the supernatant by aspiration with a pipette
- Resuspend the beads in 400 uL of PBS
- Repeat above wash steps a further 3x
- Resuspend the samples in 50 uL of PBS

### 2^{nd} Trypsin Digest

- Add 50 uL of trypsin to each sample above, briefly vortex
- Incubate tubes in oven at 37°C for 2 hours, vortex briefly approx every 30 minutes
- Take samples out of 37°C oven and add 1ml of PBS
- Spin samples at 3000rpm for 5 minutes (Pico microfuge)
- Remove trypsin supernatant leaving 100 uL of cell suspension.

### 2^{nd} secondary antibody blocking step

- Dilute 3 uL non-biotinylated polyclonal chicken anti-mouse antibody to 120 uL donkey serum
- Incubate at room temperature for 10 minutes
- Add 1mL of PBS to each tube to wash the sample
- Spin samples at 3000rpm for 5 minutes (Pico microfuge)
- Remove 1mL of supernatant leaving 100u1 in tube
- Repeat above wash steps a further 2 times
- (Cells should be left in 100 uL of PES)
- Add 6uL anti-folate binding protein antibody (LK26) to 60 uL donkey serum
- Add 10uL LK26/donkey serum to each sample
- Incubate tubes at room temperature for one hour on a shaker

### Dynabeads M-280 Streptavidin Preparation

- Add 30uL (5ul per sample)of Dynabeads to a 1.5mL centrifuge tube.
- Add 200uL 2 x binding Buffer (10mM Tris-HCl pH 7.5, 1mM EDTA, 2M NaCl on the bench) to tube
- Place the tube on the magnet for 1-2 min without removing the tube from the magnet during the separation process.
- Remove the supernatant by aspiration with a pipette while the tube remains on the magnet.
- Remove the tube from the magnet and add 200 uL 2x binding buffer along the inside of the tube where the Dynabeads are collected.
- Repeat above wash steps a further 3x times and at the last wash resuspend the sample in 60 ul of 1 x Binding buffer (ie add 30ul of 2x binding buffer and 30uL of milliQ H₂O to make the binding buffer 1x)
- Add 6uL (1uL per sample) of biotinylated polyclonal antibody polyclonal chicken anti- mouse antibody to the magnetic beads and then vortex briefly
- Incubate tubes at room temperature for one hour on a shaker

### Washing samples

- Add 1mL of PBS to each tube to wash the sample
- Spin samples at 3000rpm for 5 minutes (Pico microfuge)
- Remove 1ml of supernatant leaving 100 uL in tube
- Repeat above wash steps a further two times

### (Cells should be left in 100 uL of PBS)

### Washing Dynabeads

- Wash the Dynabeads while the samples above are spinning after their 2^{nd} wash
- Add 200 uL of PBS to the magnetic Dynabeads
- Place the tube on the magnet for 1-2 minutes, do not remove the tube from the magnet during the separation process
- Remove the supernatant by aspiration with a pipette while the tube remains on the magnet.
- Resuspend the beads in 200 uL of PBS
- Repeat above wash steps a further further three times.
- Resuspend the magnetic Dynabeads in 60ul (10ul for each sample) of Donkey Serum
- Add 10 uL of magnetic Dynabeads above to each sample and vortex briefly
- Incubate tubes at room temperature for one hour on a shaker

### Washing samples

- Take samples off the shaker, vortex briefly and add 300 uL of PBS to the samples
- Place the tube on the magnet for 1-2 minutes, do not remove the tube from the magnet during the separation process
- Remove the supernatant by aspiration with a pipette while the tube remains on the magnet.
- Resuspend the beads in 400 uL of PBS
- Repeat above wash steps a further 3x
- Resuspend the samples in 100 uL of PBS
- Store samples in 4°C fridge overnight.

### EXAMPLE 5

### Optimization of gradient centrifugation conditions

The aim of these experiments was to investigate if a non-simultaneous, discontinuous gradient can be used for enrichment of fetal cells. An additional aim was to investigate if a non-simultaneous, discontinuous gradient improves yield and/or fold-enrichment and to investigate if repeating the gradient centrifugation process increases the fold enrichment of fetal cells from pap smears without significantly reducing the yield.

Fetal cells enriched by gradient centrifugation and MACS appear to be more resistant to cell lysis than single buccal cells run in parallel. One reason for lysis resistance may be that the simultaneous, continuous gradient centrifugation step may harden the cell membrane due to being spun at 22000g. Furthermore this step may cause significant cell loss due to weak and partially degraded cells lysing and the retrieved cells may be a population of cells more resistant to lysis. Therefore it may be possible to increase the yield of fetal cells as well as the susceptibility to lysis by centrifuging in pre-prepared gradients.

The contaminating maternal cells present after gradient centrifugation may not actually have a similar density as the fetal cells and may be due to overloading of the gradient column. Therefore it may be possible to further enrich the fetal cells without significant fetal cell loss by performing a second centrifugation step, which would retrieve the majority of fetal cellswhilst allowing contaminating maternal cells to layer out at their actual density.

### Methods

### Experiment 1: Simultaneous Vs non-simultaneous continuous gradient centrifugation

Two pap smear samples were split into 2x200 ul aliquots and digested with 200 uL Trypsin for 2 hours at 37 deg to produce a single cell suspension. Sample was diluted with 800uL of PBS, centrifuged down (3000 rpm, 5 min, microfuge) and supernatant removed. One cell pellet from each patient was layered on top of 3mL of 2:1 Percoll:PBS and simultaneously centrifuged at 22,000 x g for 30 minutes. A further 2 tubes of percoll:PBS were centrifuged without sample added. Remaining cell pellets were then layered on top of pre-prepared gradient and centrifuged at 1500 rpm for 10 minutes in the plate centrifuge. Fetal bands were isolated and immunohistochemistry was performed using LK26 and human placental lactogen antibodies using previously described protocols.

### Experiment 2: Simultaneous, continuous Vs non-simultaneous, non continuous gradient centrifugation

3 Pap smears were split into 4 x 200 uL alquots and digested with 200 uL Trypsin for 2 hours at 37 deg to produce a single cell suspension. Sample was diluted with 800uL PBS, centrifuged down (3000 rpm, 5 min, microfuge) and supernatant removed.

2 samples from each patient were simultaneously centrifuged in a non-preformed gradient to create a continuous gradient as above. Fetal bands were isolated as above. One of these samples from each patient was re-centrifuged in a simultaneous, continuous gradient and the fetal band again isolated.

2 samples from each patient were centrifuged by layering on top of a non-continuous gradient and spinning at 1500 rpm for 10 minutes (plate centrifuge). This gradient consisted of 3 layers: 1.131g/mL (neat percoll), 1.05g/mL (1mL percoll + 1.62 mL PBS) and 1.02g/mL (1 mL percoll + 5.55 mL PBS). These samples were run with density marker beads to visualize densities. Cells with densities between 1.033g/mL and 1.121g/mL (in between orange and red marker beads and consisting of the entire 1.05g/mL band as well as the 1.05/1.131g/mL interface) were collected. One of these samples from each patient was re-centrifuged in another non-simultaneous, non-continuous gradient and the fetal band isolated.

Immunohistochemistry was performed on isolated cells using antibodies to LK26 and human placental lactogen using previously described protocols.

### Results

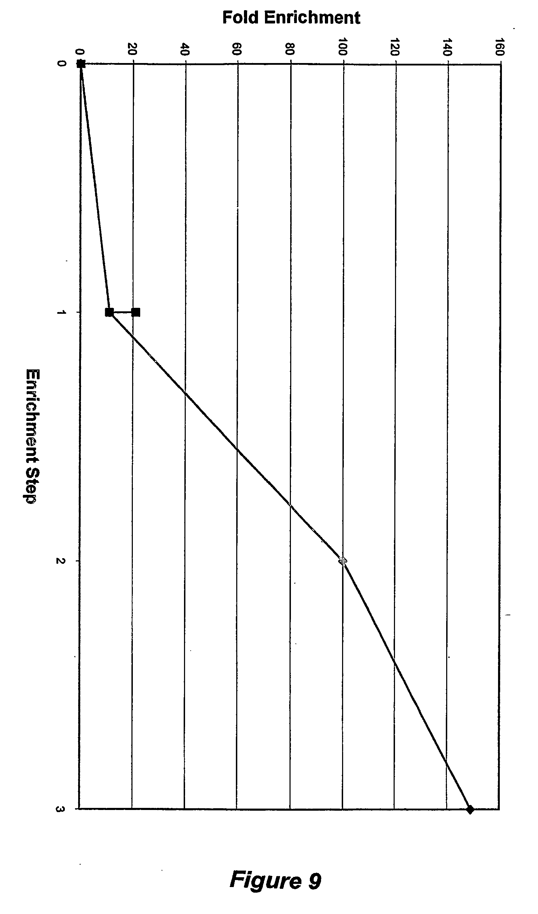

The results are summarized in Tables 6 and 7 and Figure 9.

Non-simultaneous gradient centrifugation provides an equivalent enrichment of fetal cells, however the total number of fetal cells and maternal cells available for analysis is increased 1.5-2 fold. This increase is most likely due to both cell types having less lysis caused by the harsh 22, 000 x g centrifugation. Furthermore this data shows the lysis of cells at 22, 000 x g centrifugation is equivalent in both fetal and maternal cell types.

In experiment 2 discontinuous, non-simultaneous gradient centrifugation again provides an equivalent enrichment of fetal cells when compared to simultaneous, continuous gradient centrifugation. Again in most samples there is less lysis of cells in the non-simultaneous gradient. By repeating the gradient centrifugation an average 1.9 fold enrichment is achieved by the second gradient, and is equivalent for both gradient methods. The average loss of fetal cells in the second discontinuous, non simultaneous gradient is 33%, compared to 55% with simultaneous, continuous gradient centrifugation. This can again be explained by cell lysis in the harsh 22, 000 x g centrifugation.

Non-simultaneous, discontinuous gradient enrichment appears to be the method of choice for enrichment of fetal cells from pap smears as it produces a population with equivalent fetal cell percentage, whilst increasing the fetal cell yield. Repeating this process is also recommended due to the level of enrichment achieved with relatively low levels of fetal cell loss. Non-simultaneous, discontinuous gradient enrichment can also be applied to a high throughput 96 well plate format using the CRS robotic system and the hydra 96 dispenser.

### EXAMPLE 5

### Complement Mediated Lysis of Maternal Cells

The aim of these experiments was to investigate the suitability of complement mediated lysis using monoclonal antibody against Epithelial specific antigens on maternal cells.

### Methods

Antibodies are bound to cells before addition of complement by adding 6uL of Anti-ESA antibody to 200 uL of pap smear samples, incubating for 2 hours at room temperature followed by centrifugation at 3000rpm (microfuge) and removal of supernatant. (repeat wash X 2)

For complement-mediated lysis, 200uL mouse complement will be added to the antibdy-bound cells, mixed and incubate at 37°C for a further 2 hours with intermittent mixing. Add 1mL PBS, centrifuge at 3000rpm and remove supernatant. Continue enrichment by Gradient centrifugation.

### Results

Preliminary experiments tested the specificity of antibodies directed to the following epithelial cell antigens.
Epithelial Membrane Antigen (EMA)
Epithelial Specific Antigen (ESA)
Cytokeratin (AE1/AE3)
Cytokeratin 7
Cytokeratin 20
Cytokeratin S, 18, 19 (Low molecular weight)(CAM 5.2)
Cytokeratin 1,2,5,10,14/15 (High Molecular Weight) (Clone 34BE12)
Cytokeratin 10,17,18 (MNF-116)
CEA (clone Col 1)
Estrogen Receptor
BCL-2
Ham56

As all anti-epithelial antibodies were raised in mouse each antibody was tested in parallel with rabbit anti human placental lactogen (plac) on both untreated and gradient enriched cells from 2 pap samples. A panel of 12 anti-epithelial antibodies was tested to determine sensitivity and specificity towards cells present in Pap smears.

A general conclusion from the results shown in Table 8 is that although some antibodies were found to have high sensitivity towards maternal epithelial cells, most antibodies also displayed some cross-reactivity towards fetal cells.

It is therefore anticipated that minimization of cross-reactive lysis of fetal cells when using complement-mediated lysis is required to selectively deplete or remove contaminating maternal epithelial cells from Pap smear and other cervical samples.

### EXAMPLE 6

### Hypotonic shock lysis of RBC contaminated Pap smear samples

### Method 1

Based on the method of Latham et al., 1996, Prenatal Diagnosis 16 813-821.
- Incubate pap sample in lysis buffer (0.1M NH₄Cl, 15mM NaHCO₃, 0.1mM Na₂EDTA) for 5 minutes at room temperature
- Centrifuge at 400g for 5 minutes at room temperature
- Resuspend in lysis buffer and incubate for 5 minutes at room temperature
- Centrifuge at 400g for 5 minutes at room temperature and resuspend in PBS (repeat wash X2)

### Method 2

Based on the method of Krabchi, et al., 2001, Clin Genet 60 145-50.
- Incubate pap sample in lysis buffer (0.075M KCl) for 5 minutes at 37°C
- Centrifuge at 400g for 5 minutes at room temperature and resuspend in PBS (repeat was X2)

### Results

Differential lysis is a very valuable and commonly used tool when isolating cell lineages from blood samples. The most common contaminating cell type in blood samples is un-nucleated red blood cells. These can be removed using hypotonic solutions (commonly 0.075M KCl our 0.1M NH₄Cl, 15mM NaHCO₃, 0.1mM Na₂EDTA) that preferentially lyse these cells. Severe RBC contamination is generally avoided in Pap smear samples, however in those Pap samples that are blood stained, hypotonic shock can be used to lyse RBCs prior to enrichment by other techniques.

Another method commonly used with maternal blood samples from pregnant women is NH₄Cl/HCO₃⁻ mediated lysis. This technique preferentially lyses adult RBCs, leaving fetal RBCs intact due to their lower carbonic anhydrase activity. This method is not routinely applicable to Pap smear samples as fetal RBCs are usually not the target cells being enriched. However, this is another method that would lyse maternal RBCs present in a Pap smear.

Physical differential lysis (*i.e*. non-antibody mediated) is very difficult in Pap smear samples due to the similar characteristics of the maternal epithelial cells and fetal placental cells. In our experiments, varying the pH conditions and salt concentrations did not preferentially lyse any of the contaminating cell types present in pap smears.

### EXAMPLE 7

### Isolation of nucleic acids from a single, enriched fetal cell

### Method 1

- Add 1ul of Lysis Buffer (200mM KOH, 50mM DTT) to each single cell in a 0.2mL PCR tube
- Incubate at 65°C for 10 minutes
- Add 1ul of Neutralization Buffer (300mM KCl, 900mM Tris-HCL pH 8.3, 200mM HCl) to each tube

### Method 2

- Add 1ul of Lysis Buffer (200mM KOH, 50mM DTT) to each single cell in a 0.2mL PCR tube
- Incubate at 65°C for 10 minutes
- Add 1ul of Neutralization Buffer (300mM KCl, 900mM Tris-HCL pH 8.3, 200mM HCl) to each tube
- Add 1ul of 30mM Mercapto-ethanol to each tube

### Method 3

- Add 1ul of Proteinase K (dilued 1:5 with 10mM Tris-HCl pH 7.5) to each single cell in a 0.2mL PCR tube
- Incubate at 56°C for 15 minutes
- Denature pK at 95°C for 2 minutes

### Method 4 (Preferred Method)

- Add 1ul of Proteinase K (dilued 1:5 with 10mM Tris-HCl pH 7.5) to each single cell in a 0.2mL PCR tube
- Incubate at 56°C for 15 minutes
- Denature pK at 95 °C for 2 minutes
- Add 1ul of Lysis Buffer (200mM KOH, 50mM DTT) to each pK lysed single cell in a 0.2mL PCR tube
- Incubate at 65°C for 10 minutes
- Add 1ul of Neutralization Buffer (300mM KCl, 900mM Tris-HCL pH 8.3, 200mM HCl) to each tube
- Add 1ul of 30mM Mercapto-ethanol to each tube

### Summary

This study has shown that serial enrichment of fetal cells from Pap smears can be performed using gradient centrifugation and antibody-mediated selection such as by MACS, both of which can be automated and applied to automated high throughput platforms to maximize cost effectiveness. Fetal cells were enriched and efficiently isolated from 90% of samples (n=10) with cell origin specifically confirmed by MFPCR. This embodiment therefore represents a substantial advance compared to prior art and confirms that non-invasive prenatal diagnosis from Pap smears has the potential to become an alternative procedure for obtaining fetal cells for subsequent genetic analysis.

Throughout this specification, the aim has been to describe the preferred embodiments of the invention without limiting the invention to any on embodiment or specific collection of features.

**Table 1**

| Pap Smear | % Fluorescent Cells | Gestation (weeks) |
|---|---|---|
| Pap 9 | 0.44 | 20 |
| Pap 11 | 0.57 | 12 |
| Pap 12 | 0.47 | 11 |
| Pap 13 | 1.60 | 21 |

**Table 2**

| Patient/ Antibody Set | Gestation | %++ Fluorescent | %Fluorescent | ++ isolations analysed by MFPCR | Total isolations analysed by MFPCR | % ++ fetal by MFPCR | % fetal by MFPCR | Total Cells/Sample | Fetal cells/Sample |
|---|---|---|---|---|---|---|---|---|---|
| 1A | 10 | 0.09 | 0.22 | 11 | 13 | 64 | 54 | 589680 | 683 |
| 1B | 10 | 0.08 | 0.29 | 4 | 6 | 50 | 67 | 372600 | 720 |
| 2A | 16 | 0.34 | 0.61 | 9 | 9 | 11 | 11 | 352512 | 237 |
| 2B | 16 | 0.26 | 0.28 | 2 | 2 | 50 | 50 | 222264 | 311 |
| 3A | 15 | 0.09 | 0.56 | 1 | 8 | 0 | 63 | 251100 | 878 |
| 3B | 15 | 0.00 | 0.84 | 0 | 10 | | 10 | 89856 | 76 |
| 4A | 21 | 0.07 | 0.08 | 4 | 4 | 50 | 50 | 1205280 | 486 |
| 4B | 21 | 0.07 | 0.09 | 7 | 7 | 43 | 43 | 998244 | 382 |
| 5A | 16 | 2.50 | 15.00 | 8 | 8 | 75 | 75 | 140400 | 15795 |
| 5B | 16 | 1.43 | 10.00 | 1 | 1 | 0 | 0 | 147420 | 0 |
| 6A | 7 | 1.27 | 1.58 | 8 | 9 | 88 | 89 | 59670 | 840 |
| 6B | 7 | 0.66 | 0.95 | 1 | 1 | 100 | 100 | 28431 | 270 |
| 7A | 23 | 3.57 | 17.86 | 12 | 13 | 92 | 92 | 66528 | 10966 |
| 7B | 23 | 2.50 | 17.50 | 3 | 7 | 100 | 100 | 97200 | 17010 |
| 8A | 13 | 0.15 | 0.43 | 5 | 5 | 60 | 60 | 394956 | 1021 |
| 8B | 13 | 0.28 | 0.59 | 5 | 5 | 60 | 60 | 233280 | 826 |
| 9A | 20 | 0.11 | 0.54 | 1 | 4 | 0 | 50 | 95256 | 257 |
| 9B | 20 | 0.05 | 0.44 | 0 | 1 | | 0 | 49248 | 0 |
| 10A | 14 | 0.21 | 0.68 | 6 | 13 | 33 | 31 | 2517480 | 5234 |
| 10B | 14 | 0.15 | 0.59 | 2 | 7 | 0 | 0 | 1652400 | 0 |
| 11A | 12 | 0.74 | 0.83 | 3 | 8 | 88 | 88 | 84240 | 614 |
| 11B | 12 | 0.51 | 0.57 | 9 | 9 | 67 | 67 | 80028 | 306 |
| 12A | 11 | 0.56 | 0.87 | 12 | 15 | 83 | 87 | 338985 | 2551 |
| 12B | 11 | 0.20 | 0.47 | 5 | 6 | 60 | 67 | 178200 | 558 |
| 13A | 21 | 0.58 | 1.19 | 8 | 11 | 75 | 82 | 65610 | 641 |
| 13B | 21 | 0.92 | 1.60 | 9 | 10 | 78 | 80 | 58968 | 756 |
| 14A | 20 | 0.39 | 0.76 | 10 | 11 | 20 | 18 | 130977 | 182 |
| 14B | 20 | 0.69 | 1.58 | 9 | 13 | 33 | 31 | 90288 | 440 |
| 15A | 31 | 6.25 | 8.13 | 9 | 9 | 78 | 78 | 4320 | 273 |
| 15B | 31 | 5.68 | 8.52 | 8 | 9 | 63 | 67 | 4752 | 270 |
| 16A | 17 | 0.14 | 0.37 | 9 | 13 | 78 | 77 | 483678 | 1392 |
| 16B | 17 | 0.07 | 0.28 | 2 | 6 | 50 | 67 | 348975 | 648 |
| 17A | 9 | 0.12 | 0.52 | 1 | 2 | 100 | 100 | 160380 | 837 |
| 17B | 9 | 0.26 | 0.70 | 4 | 9 | 50 | 56 | 198099 | 765 |
| 18A | 13 | 1.38 | 3.13 | 1 | 2 | 100 | 100 | 29376 | 918 |
| 18B | 13 | 1.10 | 2.39 | 8 | 10 | 25 | 20 | 29376 | 140 |
| 19A | 12 | 0.63 | 1.75 | 4 | 5 | 0 | 0 | 60021 | 0 |
| 19B | 12 | 0.66 | 1.15 | 3 | 5 | 0 | 0 | 61236 | 0 |
| 20A | 15 | 0.04 | 0.12 | 2 | 2 | 0 | 0 | 205821 | 0 |
| 20B | 15 | 0.08 | 0.15 | 1 | 1 | 0 | 0 | 214326 | 0 |
| 21A | 25 | 0.50 | 0.96 | 3 | 4 | 0 | 0 | 69984 | 0 |
| 21B | 25 | 0.35 | 0.93 | 4 | 5 | 0 | 0 | 69984 | 0 |
| 22A | 15 | 0.18 | 0.62 | 6 | 8 | 33 | 25 | 231012 | 358 |
| 22B | 15 | 0.17 | 0.41 | 4 | 4 | 0 | 0 | 254826 | 0 |
| 23A | 12 | 0.43 | 1.52 | 3 | 3 | 67 | 67 | 112266 | 1134 |
| 23B | 12 | 0.14 | 0.33 | 3 | 3 | 0 | 0 | 112860 | 0 |
| 24A | 19 | 1.12 | 1.81 | 5 | 6 | 100 | 100 | 50787 | 918 |
| 24B | 19 | 0.98 | 2.15 | 3 | 4 | 100 | 100 | 41472 | 891 |
| 25A | 27 | 0.16 | 0.38 | 3 | 4 | 33 | 25 | 215622 | 203 |
| 25B | 27 | 0.15 | 0.27 | 3 | 3 | 0 | 0 | 192510 | 0 |
| 26A | 12 | 0.68 | 1.73 | 6 | 6 | 0 | 0 | 39690 | 0 |
| 26B | 12 | 0.65 | 1.74 | 6 | 6 | 0 | 0 | 18590 | 0 |
| 27A | 12 | 4.17 | 8.33 | 0 | 0 | | | 1944 | 0 |
| 27B | 12 | 4.00 | 7.00 | 2 | 2 | 50 | 50 | 2700 | 95 |
| 28A | 13 | 0.69 | 0.89 | 4 | 7 | 0 | 0 | 123120 | 0 |
| 28B | 13 | 0.82 | 1.08 | 10 | 11 | 20 | 18 | 123930 | 243 |
| 29A | 15 | 1.09 | 1.89 | 2 | 2 | 0 | 0 | 18590 | 0 |
| 29B | 15 | 0.81 | 1.41 | 5 | 6 | 100 | 100 | 20048 | 284 |
| 30A | 18 | 1.06 | 2.13 | 9 | 10 | 11 | 10 | 43200 | 92 |
| 30B | 18 | 0.67 | 1.01 | 8 | 10 | 13 | 10 | 48114 | 49 |
| 31A | 20 | 0.00 | 1.17 | 0 | 2 | | 50 | 16200 | 95 |
| 31B | 20 | 0.33 | 0.83 | 1 | 2 | 0 | 0 | 16200 | 0 |
| 32A | 18 | 0.00 | 1.74 | 0 | 1 | | 100 | 7776 | 135 |
| 32B | 18 | 0.00 | 0.00 | 0 | 0 | | | 7776 | 0 |

**Table 3**

| | High Level Fluorescence | High and Low Level Fluorescence |
|---|---|---|
| Antibody Set A | 54 | 53 |
| Antibody Set B | 41 | 40 |

**Table 4**

| | | Initial Sample | | |
|---|---|---|---|---|
| Pap | Gestation | Blood | Cells (Opaque) | Clumps |
| 450 | 15 | ++ | ++ | ++ |
| 429 | 11 | + | + | ++ |
| 412 | 14 | - | +++ | + |
| 369 | 9 | + | + | - |
| 334 | 12 | - | - | - |
| 321 | 12 | ++ | ++ | ++ |
| 310 | 13 | + | ++ | ++ |
| 300 | 12 | - | + | + |
| 294 | 7 | - | ++ | + |
| 274 | 9 | - | ++ | + |

**Table 5**

| | Initial | Gradient | | | Primary 1 | | | | Primary 2 | | | |
|---|---|---|---|---|---|---|---|---|---|---|---|---|
| | % Fetal | % Fetal | Fold Enrich | % Loss | % Fetal | Fold Enrich | % Loss | Cells analysed | % Fetal | Fold Enrich | % Loss | Cells analysed |
| 450 | 0.431 | 1.672 | 3.9 | 84 | N/A | N/A | N/A | 0 | 63 | 145 | 91 | 8 |
| 429 | 0.624 | 9.804 | 15.7 | 76 | N/A | N/A | N/A | 0 | 40 | 64 | 97 | 5 |
| 412 | 0.236 | 1.838 | 7.8 | 84 | N/A | N/A | N/A | 0 | 70 | 297 | 89 | 10 |
| 369 | 3.735 | 6.696 | 1.8 | 72 | 7 | 1.9 | 97 | 14 | 17 | 4.5 | 97 | 6 |
| 334 | 2.451 | 16.741 | 6.8 | 69 | 50 | 20 | 98 | 2 | 43 | 17 | 94 | 7 |
| 321 | 0.473 | 5.395 | 11.4 | 80 | 64 | 135 | 94 | 22 | 29 | 60 | 99 | 7 |
| 310 | 0.339 | 5.848 | 17.3 | 60 | 0 | 0.0 | 100 | 5 | 50 | 147 | 85 | 6 |
| 300 | 1.237 | 9.259 | 7.5 | 50 | 0 | 0.0 | 100 | 2 | 0 | 0 | 100 | 3 |
| 294 | 0.139 | 2.315 | 16.7 | 72 | 33 | 240 | 97 | 3 | 50 | 360 | 94 | 4 |
| 274 | 0.295 | 2.274 | 7.7 | 65 | 20 | 68 | 98 | 5 | 23 | 78 | 78 | 39 |

**Table 6**

| Pap/method | No. Fluorescent cells | No. Total cells | % Fluorescent cells |
|---|---|---|---|
| 566 (simultaneous) | 14 | 47 | 30% |
| 566 (non-simultaneous) | 22 | 91 | 24% |
| 567 (simultaneous) | 9 | 69 | 13% |
| 567 (non-simultaneous) | 14 | 101 | 14% |

**Table 7**

| Pap/method | No. Fluorescent cells | No. Total cells | % Fluorescent cells | Fold enrichment | Fetal cell loss |
|---|---|---|---|---|---|
| 543 (1 X Discontinuous) | 45 | 176 | 26% | | |
| 543 (2 X Discontinuous) | 33 | 60 | 55% | 2.1 | 27% |
| 543 (1 X Continuous) | 36 | 163 | 22% | | |
| 543 (2 X Continuous) | 8 | 17 | 47% | 2.1 | 78% |
| 544 (1 X Discontinuous) | 37 | 198 | 19% | | |
| 544 (2 X Discontinuous) | 26 | 82 | 32% | 1.7 | 30% |
| 544 (1 X Continuous) | 34 | 176 | 19% | | |
| 544 (2 X Continuous) | 16 | 48 | 33% | 1.7 | 53% |
| 545 (1 X Discontinuous) | 220 | 4000 | 5.5% | | |
| 545 (2 X Discontinuous) | 130 | 1500 | 8.7% | 1.6 | 41% |
| 545 (1 X Continuous) | 120 | 5000 | 2.4% | | |
| 545 (2 X Continuous) | 80 | 2000 | 4% | 1.7 | 34% |

**Table 8**

| | | | Antibody reactivity (cells) | | |
|---|---|---|---|---|---|
| Pap | Ab | Treat | Epithelial | Epithelial+plac | plac |
| 446 | EMA | Neat | 2 | 9 | 13 |
| 447 | EMA | Neat | 3 | 9 | 12 |
| 440 | ESA | Grad | 1 | 6 | 0 |
| 443 | ESA | Grad | 1 | 5 | 1 |
| 452 | CAM5.2 | Neat | 15 | 8 | 0 |
| 452 | CAM5.2 | Grad | 3 | 9 | 1 |
| 200 | CAM5.2 | Neat | 11 | 6 | 0 |
| 200 | CAM5.2 | Grad | 2 | 15 | 3 |
| 452 | CK20 | Neat | 18 | 13 | 0 |
| 452 | CK20 | Grad | 6 | 5 | 0 |
| 200 | CK20 | Neat | 14 | 8 | 0 |
| 200 | CK20 | Grad | 1 | 9 | 2 |
| 452 | CK7 | Neat | 19 | 11 | 0 |
| 452 | CK7 | Grad | 1 | 4 | 0 |
| 200 | CK7 | Neat | 11 | 7 | 0 |
| 200 | CK7 | Grad | 5 | 4 | 0 |
| 452 | AE1\AE3 | Neat | 13 | 11 | 0 |
| 452 | AE1\AE3 | Grad | S | 8 | 0 |
| 200 | AE1\AE3 | Neat | S | 5 | 2 |
| 200 | AE1\AE3 | Grad | 13 | 4 | 1 |
| 475 | 34BE12 | Neat | 50-65% | 6 | 0 |
| 475 | 34BE12 | Grad | 8 | 6 | 4 |
| 217 | 34BE12 | Neat | 50-65% | 8 | 0 |
| 217 | 34BE12 | Grad | 15 | 10 | 1 |
| 475 | ER | Neat | 20% | 9 | 0 |
| 475 | ER | Grad | 7 | 8 | 2 |
| 217 | ER | Neat | 10% | 13 | 2 |
| 217 | ER | Grad | 12 | 13 | 3 |
| 195 | BCL2 | Neat | 11 | 8 | 0 |
| 195 | BCL2 | Grad | 13 | 6 | 0 |
| 204 | BCL2 | Neat | 6 | 2 | 1 |
| 204 | BCL2 | Grad | 5 | 4 | 0 |
| 195 | Ham5.6 | Neat | 8 | 4 | 0 |
| 195 | Ham5.6 | Grad | 13 | 11 | 0 |
| 204 | Ham5.6 | Neat | 1 | 1 | 1 |
| 204 | Ham5.6 | Grad | 2 | 3 | 0 |
| 217 | CEA | Neat | 23 | 10 | 5 |
| 217 | CEA | Grad | 7 | 8 | 2 |
| 475 | CEA | Neat | 18 | 5 | 0 |
| 475 | CEA | Grad | 6 | 4 | 1 |
| 217 | MNF116 | Neat | 24 | 15 | 4 |
| 217 | MNF116 | Grad | 12 | 12 | 2 |
| 475 | MNF116 | Neat | 9 | 5 | 0 |
| 475 | MNF116 | Grad | 4 | 3 | 0 |

## Claims

1. A method of fetal cell enrichment from a cervical sample that includes at least one step selected from:
(i) enriching one or more fetal cells according to density;
(ii) enriching one or more fetal cells by charge flow separation; and/or
(iii) enriching one or more fetal cells by differential cell lysis;
and at least one further step of:
(iv) enriching one or more fetal cells by negatively selecting maternal cells using at least one antibody that binds a maternal cell antigen; and/or
(v) enriching one or more fetal cells by positively selecting said one or more fetal cells using at least one antibody that binds a fetal cell antigen.

2. A method of fetal cell enrichment from a Pap smear that includes at least one step selected from:
(i) enriching one or more fetal cells according to density;
(ii) enriching one or more fetal cells by charge flow separation; and/or
(iii) enriching one or more fetal cells by differential cell lysis;
(iv) enriching one or more fetal cells by negatively selecting maternal cells using at least one antibody that binds a maternal cell antigen; and/or
(v) enriching one or more fetal cells by positively selecting said one or more fetal cells using at least one antibody that binds a fetal cell antigen.

3. The method of Claim 1 or Claim 2, wherein said at least one step is enriching said fetal cell according to density.

4. The method of Claim 3, wherein step (i) includes sequential density enrichment steps.

5. The method of Claim 3, wherein said fetal cell is enriched as having a density greater than a maternal cell present in said cervical sample.

6. The method of Claim 5, wherein the fetal cell has a density in the range 1.033-1.142 g/mL

7. The method of Claim 6, wherein the fetal cell has a density in the range 1.033-1.131 g/mL or in the range 1.05-1.131 g/mL.

8. The method of Claim 3, wherein fetal cell enrichment is performed using a discontinuous density gradient or a continuous density gradient.

9. The method of Claim 8, wherein the discontinuous density gradient is a preformed or non-simultaneously formed density gradient.

10. The method of Claim 3, wherein prior to density gradient enrichment, the Pap smear or cervical sample is subjected to differential lysis to remove or otherwise deplete contaminating maternal red blood cells and thereby enrich said one more fetal cells.

11. The method of Claim 1 or Claim 2, including the step of positively selecting fetal cells using at least one antibody that binds a fetal cell antigen.

12. The method of Claim 11, wherein a fetal cell antigen is selected from the group consisting of: human placental lactogen; human chorionic gonadatrophin; human placental alkaline phosphatase and folate binding protein (LK26).

13. The method of Claim 11 or Claim 12 performed using fluorescence activated cell sorting (FACS) and/or magnetic activated cell sorting (MACS).

14. The method of Claim 13, wherein MACS is performed using the sequential steps of:
(a) enriching one or more fetal cells that express a first fetal cell antigen; and
(b) further enriching the one or more fetal cells from step (a) that express a second fetal cell antigen.

15. The method of Claim 14, wherein the first fetal cell antigen is human placental lactogen and the second fetal cell antigen is folate binding protein.

16. A method of isolating a nucleic acid including the steps of:
(i) enriching one or more fetal cells according to Claim 1 or Claim 2; and
(ii) isolating a nucleic acid from the fetal cells enriched at step (i).

17. A method of nucleic acid sequence amplification including the steps of:
(i) isolating a nucleic acid according to Claim 16; and
(ii) subjecting the nucleic acid isolated at step (i) to a nucleic acid sequence amplification technique to thereby produce one or more amplification products.

18. A method of genetic analysis of one or more fetal cells including the steps of:
(i) enriching one or more fetal cells according to Claim 1 or Claim 2; and
(ii) performing a genetic analysis of a nucleic acid sample isolated from the fetal cells enriched at step (i).

19. The method of Claim 18 wherein the genetic analysis is forensic analysis, pre-implantation genetic diagnosis (PGD), chromosomal analysis and/or sex determination.

20. The method of Claim 18, wherein the genetic analysis is of a single fetal cell enriched from a Pap smear.

21. The method of Claim 18 wherein step (ii) includes nucleic acid sequence amplification of the nucleic acid sample.

22. The method of Claim 17 or Claim 21, wherein the nucleic acid sequence amplification technique is PCR, multiplex PCR or fluorescence multiplex PCR..

## Patentansprüche

1. Verfahren zur Anreicherung fötaler Zellen aus einer Gebärmutterhalsprobe, das zumindest einen aus den folgenden Schritten ausgewählte Schritt umfasst:
(i) Anreichern einer oder mehrerer fötaler Zellen nach Dichte;
(ii) Anreichern einer oder mehrerer fötaler Zellen durch Ladungsfluss-Separation; und/oder
(iii) Anreichern einer oder mehrerer fötaler Zellen durch Differential-Zellyse;
und zumindest einen weiteren der folgenden Schritte:
(iv) Anreichern einer oder mehrerer fötaler Zellen durch negative Selektion von Mutterzellen unter Verwendung zumindest eines Antikörpers, der ein Mutterzellen-Antigen bindet; und/oder
(v) Anreichern einer oder mehrerer fötaler Zellen durch positive Selektion von Mutterzellen unter Verwendung zumindest eines Antikörpers, der ein Fötalzellen-Antigen bindet.

2. Verfahren zur Anreicherung fötaler Zellen aus einem Pap-Abstrich, das zumindest einen aus den folgenden Schritten ausgewählten Schritt umfasst:
(i) Anreichern einer oder mehrerer fötaler Zellen nach Dichte;
(ii) Anreichern einer oder mehrerer fötaler Zellen durch Ladungsfluss-Separation; und/oder
(iii) Anreichern einer oder mehrerer fötaler Zellen durch Differential-Zellyse;
(iv) Anreichern einer oder mehrerer fötaler Zellen durch negative Selektion von Mutterzellen unter Verwendung zumindest eines Antikörper, der ein Mutterzellen-Antigen bindet; und/oder
(v) Anreichern einer oder mehrerer fötaler Zellen durch positive Selektion von Mutterzellen unter Verwendung zumindest eines Antikörpers, der ein Fötalzellen-Antigen bindet.

3. Verfahren nach Anspruch 1 oder Anspruch 2, wobei dieser zumindest eine Schritt die fötale Zelle nach Dichte anreichert.

4. Verfahren nach Anspruch 3, wobei Schritt (i) sequenzielle Dichteanreicherungsschritte umfasst.

5. Verfahren nach Anspruch 3, wobei die fötale Zelle so angereicht wird, dass sie eine Dichte hat, die größer ist als die einer in der Gebärmutterhalsprobe vorhandenen Mutterzelle.

6. Verfahren nach Anspruch 5, wobei die fötale Zelle eine Dichte in dem Bereich von 1,033-1,142 g/ml hat.

7. Verfahren nach Anspruch 6, wobei die fötale Zelle eine Dichte in dem Bereich von 1,033-1,131 g/ml oder in dem Bereich von 1,05-1,131 g/ml hat.

8. Verfahren nach Anspruch 3, wobei die Anreicherung der fötalen Zelle unter Verwendung eines diskontinuierlichen Dichtegradienten oder eines kontinuierlichen Dichtegradienten durchgeführt wird.

9. Verfahren nach Anspruch 8, wobei der diskontinuierliche Dichtegradient ein vorgeformter oder nicht simultan geformter Dichtegradient ist.

10. Verfahren nach Anspruch 3, wobei vor der Dichtegrad-Anreicherung der Pap-Abstrich oder die Gebärmutterhaisprobe einer Differential-Lyse unterzogen werden, um kontaminierende maternale rote Blutzellen zu beseitigen oder anderweitig zu vermindern und **dadurch** die eine oder mehreren fötalen Zellen anzureichern.

11. Verfahren nach Anspruch 1 oder Anspruch 2, umfassend den Schritt der positiven Selektion fötaler Zellen unter Verwendung zumindest eines Antikörpers, der ein fötales Zellantigen bindet.

12. Verfahren nach Anspruch 11, wobei ein fötales Zellantigen aus der Gruppe ausgewählt wird, die aus humanen Plazentalaktogenen; humanem chorionischen Gonadatrophin; Humanplazenta-Alkaliphosphatase und Folat-Bindeprotein (LK26) besteht.

13. Verfahren nach Anspruch 11 oder Anspruch 12, das durchgeführt wird unter Anwendung einer fluoreszenzaktivierten Zellsortierung (FACS) und /oder einer magnetaktivierten Zellsortierung (MACS).

14. Verfahren nach Anspruch 13, wobei die MACS durchgeführt wird unter Anwendung der sequenziellen Schritte:
(a) Anreichern einer oder mehrerer fötaler Zellen, die ein erstes fötales Zellantigen zeigen; und
(b) weiteres Anreichern der einen oder mehreren fötalen Zellen aus Schritt (a), die ein zweites fötales Zellantigen zeigen.

15. Verfahren nach Anspruch 14, wobei das erste fötale Zellantigen ein Humanplazenta-Laktogen und das zweite fötales Zellantigen ein Folat-Bindeprotein ist.

16. Verfahren zum Isolieren von Nukleinsäure, umfassend die Schritte:
(i) Anreichern einer oder mehrerer fötaler Zellen gemäß Anspruch 1 und Anspruch 2; und
(ii) Isolieren einer Nukleinsäure aus den in Schritt (i) angereicherten fetalen Zellen.

17. Verfahren zum Verstärken einer Nukleinsäuresequenz, umfassend die Schritt:
(i) Isolieren einer Nukleinsäure gemäß Anspruch 16; und
(ii) Unterziehen der in Schritt (i) isolierten Nukleinsäure einem Nukleinsäute-Verstärkungsverfahren, um **dadurch** eines oder mehrere Verstärkungsprodukte zu bilden.

18. Verfahren zur Genanalyse einer oder mehrerer fötaler Zellen, umfassend die Schritte:
(i) Anreichern einer oder mehrerer fötaler Zellen gemäß Anspruch 1 oder Anspruch 2; und
(ii) Durchführen einer Genanalyse einer aus den in Schritt (i) angereicherten fötalen Zellen isolierten Nukleinsäureprobe.

19. Verfahren nach Anspruch 18, wobei die Genanalyse eine forensische Analyse, Präimplantations-Gendiagnose (PGD), Chromosomenanalyse und/oder Geschlechtsbestimmung ist.

20. Verfahren nach Anspruch 18, wobei die Genanalyse die Analyse einer einzigen angereicherten fötalen Zelle aus einem Pap-Abstrich ist.

21. Verfahren nach Anspruch 18, wobei Schritt (ii) eine Nukleinsäuresequenz-Verstärkung der Nukleinsäureprobe ist.

22. Verfahren nach Anspruch 17 oder Anspruch 21, wobei das Nukleinsäuresequenz-Verstärkungsverfahren eine PCR, Multiplex-PCR oder Fluoreszenz-Multiplex-PCR ist.

## Revendications

1. Procédé d'enrichissement de cellules foetales provenant d'un échantillon d'utérus comportant au moins l'une des étapes choisies parmi les étapes consistant à :
(i) enrichir au moins une cellule foetale concernant sa densité,
(ii) enrichir au moins une cellule foetale par séparation de charge d'écoulement et/ ou
(iii) enrichir au moins une cellule foetale par lyse différentielle de cellules,
et au moins une autre étape choisie parmi les étapes consistant à :
(iv) enrichir au moins une cellule foetale par sélection négative des cellules maternelles en utilisant au moins un anticorps qui lie un antigène des cellules maternelles, et/ou
(v) enrichir au moins une cellule foetale par sélection positive de cette ou de ces cellules foetales en utilisant au moins un anticorps qui lie un antigène des cellules foetales.

2. Procédé d'enrichissement de cellule foetale provenant d'un frottis Pap qui comporte au moins une étape choisie parmi les étapes consistant à :
(i) enrichir au moins une cellule foetale concernant la densité,
(ii) enrichir au moins une cellule foetale par séparation de charge découlement et/ ou
(iii) enrichir au moins une cellule foetale par lyse différentielle de cellules,
(iv) enrichir au moins une cellule foetale par sélection négative des cellules maternelles en utilisant au moins un anticorps qui lie un antigène des cellules maternelles, et/ou
(v) enrichir au moins une cellule foetale par sélection positive de cette ou de ces cellules foetales en utilisant au moins un anticorps qui lie un antigène des cellules foetales.

3. Procédé conforme à la revendication 1 ou 2,
selon lequel
l'étape est une étape d'enrichissement de la cellule foetale concernant sa densité.

4. Procédé conforme à la revendication 3,
selon lequel
l'étape (i) renferme des étapes d'enrichissement concernant la densité séquentielles.

5. Procédé conforme à la revendication 3,
selon lequel
la cellule foetale est enrichie de sorte qu'elle ait une densité supérieure à celle d'une cellule maternelle présente dans l'échantillon d'utérus.

6. Procédé conforme à la revendication 5,
selon lequel
la cellule foetale présente une densité comprise dans la plage de 1,033-1,142 g/mL.

7. Procédé conforme à la revendication 6,
selon lequel
la cellule foetale présente une densité comprise dans la plage de 1,033-1,131 g/mL ou dans la plage de 1,05-1,131 g/mL.

8. Procédé conforme à la revendication 1,
selon lequel
l'enrichissement de la cellule foetale et mise en oeuvre en utilisant un gradient de densité discontinu ou un gradient de densité continu.

9. Procédé conforme à la revendication 8,
selon lequel
le gradient de densité discontinu est un gradient de densité formé préalablement ou de manière non simultanée.

10. Procédé conforme à la revendication 3,
selon lequel
avant l'enrichissement par gradient de densité, l'échantillon de frottis Pap ou d'utérus est soumis à une lyse différentielle pour éliminer ou tout du moins réduire les globules rouges maternels contaminants et enrichir ainsi cette ou ces cellule(s) foetale(s).

11. Procédé conforme à la revendication 1 ou 2, comprenant l'étape consistant à effectuer une sélection positive des cellules foetales en utilisant au moins un anticorps qui lie un antigène des cellules foetales.

12. Procédé conforme à la revendication 11,
selon lequel
l'antigène des cellules foetales est choisi dans le groupe formé par le lactogène placentaire humain, la gonadotrophine chorionique humaine, la phosphatase alcaline placentaire humaine et les protéines liant les folates (LK26).

13. Procédé conforme à la revendication 11 ou 12, mise en oeuvre en utilisant le triage de cellules activé par fluorescence (FACS) et/ou le triage de cellules par activation magnétique (MACS).

14. Procédé conforme à la revendication 13,
selon lequel
le MACS est mis en oeuvre en utilisant les étapes séquentielles consistant à :
(a) enrichir au moins une cellule foetale qui exprime un premier antigène des cellules foetales, et
(b) enrichir ensuite la ou les cellule(s) foetale(s) provenant de l'étape (a) qui exprime(nt) un second antigène des cellules foetales.

15. Procédé conforme à la revendication 14,
selon lequel
le premier antigène des cellules foetales est le lactogène placentaire humain et le second antigène des cellules foetales est une protéine liant les folates.

16. Procédé d'isolation d'un acide nucléique comportant les étapes consistant à :
(i) enrichir au moins une cellule foetale selon la revendication 1 ou 2, et
(ii) isoler un acide nucléique provenant des cellules foetales enrichies à l'étape (i).

17. Procédé d'amplification d'une séquence d'acide nucléique comportant les étapes consistant à :
(i) isoler un acide nucléique selon la revendication 16, et
(ii) soumettre l'acide nucléique isolé à l'étape (i) à une technique d'amplification d'une séquence d'acide nucléique pour obtenir au moins un produit d'amplification.

18. Procédé d'analyse génétique d'au moins une cellule foetale comportant les étapes consistant à :
(i) enrichir au moins une cellule foetale selon la revendication 1 ou 2, et
(ii) mettre en oeuvre une analyse génétique d'un échantillon d'acide nucléique isolé à partir des cellules foetales enrichies à l'étape (i).

19. Procédé conforme à la revendication 18,
selon lequel
l'analyse génétique est une analyse forensique, un diagnostic génétique de pré-implantation (PGD), une analyse chromosomique et/ou une détermination du sexe.

20. Procédé conforme à la revendication 18,
selon lequel
l'analyse génétique est l'analyse d'une cellule foetale unique enrichie provenant d'un frottis Pap.

21. procédé conforme à la revendication 18,
selon lequel
l'étape (ii) comporte l'amplification d'une séquence d'acide nucléique de l'échantillon d'acide nucléique.

22. Procédé conforme à la revendication 17 ou 21
selon lequel
la technique d'amplification de la séquence d'acide nucléique est la PCR, la PCR multiplexe, ou la PCR multiplex par fluorescence.
